# EUROPEAN PATENT APPLICATION

(11) **EP 2 818 164 A1**
(43) Date of publication of application: **31.12.2014**
(21) Application number: 14170043.5
(22) Date of filing: 09.02.2010
(51) Int. Cl.: A61K 31/33, A61K 31/40, A61K 31/4035, A61K 31/42, A61K 31/44, A61K 31/4409, A61K 31/495, A61K 31/496, A61K 31/70, A61P 31/06

(54) **Methods of using and compositions comprising PDE4-modulators for treatment, prevention and management of tuberculosis**

(30) Priority: 10.02.2009 US 151467 P
(62) Divisional of application: 10704460.4
(71) Applicant: CELGENE CORPORATION, Summit, NJ 07901 (US)
(72) Inventor: Zeldis, Jerome, Princeton, NJ 08540 (US); Kaplan, Gilla, New York, NY 10022 (US)
(74) Representative: Jones Day

(57) **Abstract**

The present invention concerns a PDE4 modulator for use in a method of treating, preventing, or managing tuberculosis, the method comprising administering to a subject a PDE4 modulator, or a pharmaceutically acceptable salt, solvate, or stereoisomer thereof, in combination with an anti-tuberculosis agent.

## Description

### 1. FIELD

Provided herein are methods of treating, preventing, and/or managing tuberculosis and related disorders, using PDE4 modulators. Pharmaceutical compositions and dosing regimens for such treatment, prevention, and/or management arc also provided herein.

### 2. BACKGROUND

### 2.1 TUBERCULOSIS

Tuberculosis (TB) is a chronic, recurrent infection by mycobacteria, most commonly in the lungs. Once infection is established, clinical TB may develop within months or may not occur for years. TB generally refers only to disease caused by *M. tuberculoses, M. Bovis,* or *M. africanum,* although other mycobacteria may cause diseases similar to TB.

TB progresses through the stages of primary or initial infection, latent or dormant infection, and recrudescent or adult-type TB. A great portion of primary TB infections go unrecognized, producing a latent or dormant infection. Primary TB may become active at any time, producing clinical signs of TB in any organ.

TB is generally treated with a chemotherapy, using bactericidal or bacteriostatic drugs. However, TB treatment is complicated, requiring 6 months or longer to clear the infection. In addition, the strains that cause TB generally include population that are resistant to the drug therapy, resulting in initial improvement followed by recurrence of the symptoms. Therefore, a need still exists for effective treatments for TB and related disorders.

### 2.2 PDE4 MODULATORS

Compounds referred to as PDE4 modulators have been synthesized and tested. These compounds potently inhibit TNF-α and IL-12 production, and exhibit modest inhibitory effects on LPS induced IL1β. L.G. Corral, et al., J. Immunol., 163: 380-386 (1999).

PDE4 is one of the major phosphodiesterase isoenzymes found in human myeloid and lymphoid lineage cells. The enzyme plays a crucial part in regulating cellular activity by degrading the ubiquitous second messenger cAMP and maintaining it at low intracellular levels. *Id.* Inhibition of PDE4 activity results in increased cAMP levels leading to the modulation of chemokines and cytokines including inhibition of TNF-α production in monocytes as well as in lymphocytes.

### 3. SUMMARY

Provided herein are methods of treating, preventing, and/or managing TB and/or other disorders, which comprise administering to a subject a therapeutically or prophylactically effective amount of a PDE4 modulator, or a pharmaceutically acceptable salt, solvate (*e*.*g*., hydrate), stereoisomer, or prodrug thereof, optionally in combination with one or more conventional TB treatments.

In one embodiment, provided herein is the use of one or more PDE4 modulators in combination with one or more chemotherapeutics or other therapies presently used to treat, prevent and/or manage TB.

Also provided herein are pharmaceutical compositions, single unit dosage forms, and kits suitable for use in treating, preventing, and/or managing TB and other disorders, which comprise a PDE4 modulator, or a pharmaceutically acceptable salt, solvate, stereoisomer, or prodrug thereof, optionally in combination with one or more other therapeutic agents.

### 4. DETAILED DESCRIPTION

Compounds provided herein can work alone or in combination with other drugs to effectively treat, prevent, and/or manage various types of TB and other related disorders. Without being limited by a particular theory, PDE4 inhibitors are believed to exert their effects by elevating intracellular cAMP levels in various leukocytes, including T cells, B cells, NK cells, neutrophils, monocytes, macrophages, basophils, eosinophils, and mast cells. PDE4 is also expressed in non-leukocytes such as endothelial cells, smooth muscle cells, fibroblasts, and neurons, which are also believed to contribute to inflammation.

The currently available anti-TB drugs work through their ability to inhibit and/or kill actively growing organisms, and thus, are less effective against non-replicating bacilli. Without being limited by a particular theory, it is believed that a strong host immune response may drive some bacilli into a non-replicating state, thereby inadvertently rendering the organisms more able to withstand the activities of anti-TB drugs. Further without being limited by a particular theory, it is believed that selective modulation of the host immune response may alter the environmental pressure on the bacilli, re-directing their physiologic state towards improved responsiveness to anti-TB drugs.

Provided herein are methods of treating, preventing, and/or managing TB and related disorders, which comprise administering to a subject a therapeutically or prophylactically effective amount of a PDE4 modulator, or a pharmaceutically acceptable salt, solvate, stereoisomer, or prodrug thereof, optionally in combination with one or more anti-TB agents or therapies.

Also provided herein are pharmaceutical compositions, single unit dosage forms, and kits, which comprise one or more PDE4 modulators, or a pharmaceutically acceptable salt, solvate, stereoisomer, or prodrug thereof, and one or more other therapeutic agents. For example, a kit may contain one or more compounds provided herein and one or more anti-TB therapeutic agents.

Particular PDE4 modulators may improve the therapeutic efficacy of conventional therapies for TB and related disorders.. Consequently, also provided herein are methods of improving therapeutic efficacy of a conventional therapy or therapeutic agent, which comprises administering to a subject who is receiving such a therapy or therapeutic agent a therapeutically or prophylactically effective amount of a PDE4 modulator, or a pharmaceutically acceptable salt, solvate, stereoisomer, or prodrug thereof.

### 4.1 DEFINITIONS

As used herein and unless otherwise indicated, the term "PDE4 modulators" encompasses small molecule drugs, e.g., small organic molecules which arc not peptides, proteins, nucleic acids, oligosaccharides or other macromolecules. In certain embodiments, the compounds inhibit TNF-α production. Compounds may also have an inhibitory effect on LPS induced IL1β and IL12. In some embodiments, the compounds are potent PDE4 inhibitors.

As used herein, and unless otherwise specified, the term "pharmaceutically acceptable salt" refers to salts prepared from pharmaceutically acceptable non-toxic acids, including inorganic acids and organic acids. Suitable non-toxic acids include inorganic and organic acids such as, but not limited to, acetic, alginic, anthranilic, benzenesulfonic, benzoic, camphorsulfonic, citric, ethenesulfonic, formic, fumaric, furoic, gluconic, glutamic, glucorenic, galacturonic, glycidic, hydrobromic, hydrochloric, isethionic, lactic, maleic, malic, mandelic, methanesulfonic, mucic, nitric, pamoic, pantothenic, phenylacetic, propionic, phosphoric, salicylic, stearic, succinic, sulfanilic, sulfuric, tartaric acid, p-toluenesulfonic and the like. In some embodiments, the salts arc hydrochloric, hydrobromic, phosphoric, and sulfuric acids.

As used herein, and unless otherwise specified, the term "solvate" means a compound provided herein or a salt thereof, that further includes a stoichiometric or non-stoichiometric amount of solvent bound by non-covalent intermolecular forces. Where the solvent is water, the solvate is a hydrate.

As used herein, and unless otherwise specified, the term "prodrug" means a derivative of a compound that can hydrolyze, oxidize, or otherwise react under biological conditions (*in vitro* or *in vivo*) to provide the compound. Examples of prodrugs include, but are not limited to, compounds that comprise biohydrolyzable moieties such as biohydrolyzable amides, biohydrolyzable esters, biohydrolyzable carbamates, biohydrolyzable carbonates, biohydrolyzable ureides, and biohydrolyzable phosphate analogues. Other examples of prodrugs include compounds that comprise -NO, -NO₂, -ONO, or -ONO₂ moieties. Prodrugs can typically be prepared using well-known methods, such as those described in Burger's Medicinal Chemistry and Drug Discovery, 172-178, 949-982 (Manfred E. Wolff ed., 5th ed. 1995), and Design of Prodrugs (H. Bundgaard ed., Elselvier, New York 1985).

As used herein, and unless otherwise specified, the terms "biohydrolyzable carbamate," "biohydrolyzable carbonate," "biohydrolyzable ureide" and "biohydrolyzable phosphate " mean a carbamate, carbonate, ureide and phosphate, respectively, of a compound that either: 1) docs not interfere with the biological activity of the compound but can confer upon that compound advantageous properties *in vivo,* such as uptake, duration of action, or onset of action; or 2) is biologically inactive but is converted *in vivo* to the biologically active compound. Examples of biohydrolyzable carbamates include, but are not limited to, lower alkylamines, substituted ethylenediamines, aminoacids, hydroxyalkylamines, heterocyclic and heteroaromatic amines, and polyether amines.

As used herein, and unless otherwise specified, the term "stereoisomer" encompasses all enantiomerically/stercomerically pure and enantiomerically/stercommerically enriched compounds provided herein.

As used herein, and unless otherwise indicated, the term "stereomerically pure" or "enantiomerically pure" means that a compound comprises one stereoisomer and is substantially free of its counter stereoisomer or enantiomer. For example, a compound is stereomerically or enantiomerically pure when the compound contains 80%, 90%, or 95% or more of one stereoisomer and 20%, 10%, or 5% or less of the counter stereoisomer. In certain cases, a compound provided herein is considered optically active or stereomerically/enantiomerically pure (*i.e*., substantially the R-form or substantially the S-form) with respect to a chiral center when the compound is about 80% ee (enantiomeric excess) or greater, preferably, equal to or greater than 90% ee with respect to a particular chiral center, and more preferably 95% ee with respect to a particular chiral center.

As used herein, and unless otherwise indicated, the term "stereomerically enriched" or "enantiomerically enriched" encompasses racemic mixtures as well as other mixtures of stereoisomers of compounds provided herein (*e.g*., R/S = 30/70, 35/65, 40/60, 45/55, 55/45, 60/40, 65/35 and 70/30).

As used herein, and unless otherwise specified, the terms "treat," "treating" and "treatment" contemplate an action that occurs while a patient is suffering from the specified disease or disorder, which reduces the severity of the disease or disorder, or retards or slows the progression of the disease or disorder.

As used herein, unless otherwise specified, the terms "prevent," "preventing" and "prevention" contemplate an action that occurs before a patient begins to suffer from the specified disease or disorder, which inhibits or reduces the severity of the disease or disorder.

As used herein, and unless otherwise indicated, the terms "manage," "managing" and "management" encompass preventing the recurrence of the specified disease or disorder in a patient who has already suffered from the disease or disorder, and/or lengthening the time that a patient who has suffered from the disease or disorder remains in remission. The terms encompass modulating the threshold, development and/or duration of the disease or disorder, or changing the way that a patient responds to the disease or disorder.

As used herein, and unless otherwise specified, the term "therapeutically effective amount" of a compound is an amount sufficient to provide a therapeutic benefit in the treatment or management of a disease or condition, or to delay or minimize one or more symptoms associated with the disease or condition. A therapeutically effective amount of a compound means an amount of therapeutic agent, alone or in combination with other therapies, which provides a therapeutic benefit in the treatment or management of the disease or condition. The term "therapeutically effective amount" can encompass an amount that improves overall therapy, reduces or avoids symptoms or causes of disease or condition, or enhances the therapeutic efficacy of another therapeutic agent.

As used herein, and unless otherwise specified, the term "prophylactically effective amount" of a compound is an amount sufficient to prevent a disease or condition, or one or more symptoms associated with the disease or condition, or prevent its recurrence. A prophylactically effective amount of a compound means an amount of therapeutic agent, alone or in combination with other agents, which provides a prophylactic benefit in the prevention of the disease. The term "prophylactically effective amount" can encompass an amount that improves overall prophylaxis or enhances the prophylactic efficacy of another prophylactic agent.

### 4.2 PDE4 MODULATORS

Compounds provided herein include racemic, stercomerically pure and stereomerically enriched PDE4 modulators, stereomerically and enantiomerically pure compounds that have selective cytokine inhibitory activities, and pharmaceutically acceptable salts, solvates, hydrates, stereoisomers, clathrates, and prodrugs thereof. In certain embodiments, compounds are known PDE4 modulators of Celgene Corporation, NJ.

Examples of PDE4 modulators include, but arc not limited to, the cyclic imides disclosed in U.S. patent nos. 5,605,914 and 5,463,063; the cycloalkyl amides and cycloalkyl nitriles of U.S. patent nos. 5,728,844, 5,728,845, 5,968,945, 6,180,644 and 6,518,281; the aryl amides (for example, an embodiment being N-benzoyl-3-amino-3-(3',4'-dimethoxyphenyl)-propanamide) of U.S. patent nos. 5,801,195, 5,736,570, 6,046,221 and 6,284,780; the imide/amide ethers and alcohols (for example, 3-phthalimido-3-(3',4'-dimethoxyphenyl)propan-1-ol) disclosed in U.S. patent no. 5,703,098; the succinimides and malcimides (for example methyl 3-(3',4',5'6'-petrahydrophthalimdo)-3-(3",4"-dimethoxyphenyl)propionate) disclosed in U.S. patent no. 5,658,940; imido and amido substituted alkanohydroxamic acids disclosed in U.S. patent no. 6,214,857 and WO 99/06041; substituted phenethylsulfones disclosed in U.S. patent nos. 6,011,050 and 6,020,358; fluoroalkoxy-substituted 1,3-dihydro-isoindolyl compounds disclosed in U.S. patent no. 7,173,058; substituted imides (for example, 2-phthalimido-3-(3',4'-dimethoxyphenyl) propane) disclosed in U.S. patent no. 6,429,221; substituted 1,3,4-oxadiazoles (for example, 2-[1-(3-cyclopentyloxy-4-methoxyphenyl)-2-(1,3,4-oxadiazole-2-yl)ethyl]-5-methylisoindoline-1,3-dione) disclosed in U.S. patent no. 6,326,388; cyano and carboxy derivatives of substituted styrenes (for example, 3,3-bis-(3,4-dimethoxyphenyl) acrylonitrile) disclosed in U.S. patent nos. 5,929,117, 6,130,226,6,262,101 and 6,479,554; isoindoline-1-one and isoindoline-1,3-dione substituted in the 2-position with an α-(3,4-disubstituted phenyl)alkyl group and in the 4- and/or 5-position with a nitrogen-containing group disclosed in WO 01/34606 and U.S. patent no. 6,667,316, for example, cyclopropyl-N-{2-[1-(3-ethoxy-4-methoxyphenyl)-2-(methylsulfonyl)ethyl]-3-oxoisoindolin-4-yl}carboxamide, cyclopropyl-N-{2-[1(S)-(3-ethoxy-4-methoxyphenyl)-2-{methylsulfonyl)ethyl]-3-oxoisoindolin-4-yl]carboxamide, and cyclopropyl-N-{2-[1(R)-(3-ethoxy-4-methoxyphenyl)-2-(methylsulfonyl)ethyl]-3-oxoisoindolin-4-yl}carboxamide; and imido and amido substituted acylhydroxamic acids (for example, (3-(1,3-dioxoisoindoline-2-yl)-3-(3-ethoxy-4-methoxyphenyl) propanoylamino) propanoate disclosed in WO 01/45702 and U.S. patent no. 6,699,899. Other PDE4 modulators include diphenylethylene compounds disclosed in U.S. patent no. 7,312,241, the contents of which are incorporated by reference herein in their entirety. Other PDE4 modulators include isoindoline compounds disclosed in U.S. patent publication no. 2006/0025457A1, published February 2, 2006 and U.S. patent no. 7,244,759. Other specific PDE4 modulators include 2-[1-(3-ethoxy-4-methoxyphenyl)-2-methylsulfonylethyl]-4-acetylaminoisoindoline-1,3-dione, and stereoisomers thereof. (+)-2-[1-(3-ethoxy-4-methoxyphenyl)-2-methylsulfonylethyl]-4-acetylaminoisoindoline-1,3-dione was disclosed in WO 03/080049. The entireties of each of the patents and patent applications identified herein are incorporated herein by reference.

Additional PDE4 modulators belong to a family of synthesized chemical compounds of which typical embodiments include 3-(1,3-dioxobenzo-[f]isoindol-2-yl)-3-(3-cyclopentyloxy-4-methoxyphenyl)propionamide and 3-(1,3-dioxo-4-azaisoindol-2-yl)-3-(3,4-dimethoxyphenyl)-propionamide.

Other PDE4 modulators belong to a class of non-polypeptide cyclic amides disclosed in U.S. patent nos. 5,698,579, 5,877,200, 6,075,041 and 6,200,987, and WO 95/01348, each of which is incorporated herein by reference. Representative cyclic amides include compounds of the formula: 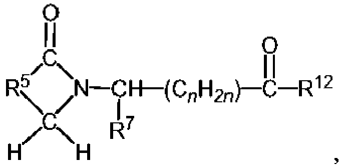 and pharmaceutically acceptable salts, solvates, hydrates, clathrates, stereoisomers, and prodrugs thereof, wherein:
n has a value of 1, 2, or 3;
R⁵ is o-phenylene, unsubstituted or substituted with 1 to 4 substituents each selected independently from the group consisting of nitro, cyano, trifluoromethyl, carbethoxy, carbomethoxy, carbopropoxy, acetyl, carbamoyl, acctoxy, carboxy, hydroxy, amino, alkylamino, dialkylamino, acylamino, alkyl of 1 to 10 carbon atoms, alkyl of 1 to 10 carbon atoms, and halo;
R⁷ is (i) phenyl or phenyl substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, (ii) benzyl unsubstituted or substituted with 1 to 3 substituents selected from the group consisting of nitro, cyano, trifluoromethyl, carbothoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, (iii) naphthyl, and (iv) benzyloxy;
R¹² is -OH, alkoxy of 1 to 12 carbon atoms, or 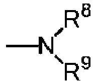
R⁸ is hydrogen or alkyl of 1 to 10 carbon atoms; and
R⁹ is hydrogen, alkyl of 1 to 10 carbon atoms, -COR¹⁰, or -SO₂R¹⁰, wherein R¹⁰ is hydrogen, alkyl of 1 to 10 carbon atoms, or phenyl.

In some embodiments, PDE4 modulator is the following compound, or a pharmaceutically acceptable salt, solvate, hydrate, clathrate, stereoisomer, or prodrug thereof:
3-phenyl-2-(1-oxoisoindolin-2-yl)propionic acid;
3-phenyl-2-(1-oxoisoindolin-2-yl)propionamide;
3-phenyl-3-(1-oxoisoindolin-2-yl)propionic acid;
3-phenyl-3-(1-oxoisoindolin-2-yl)propionamide;
3-(((4-methoxyphenyl)-3-(1-oxisoindolin-yl)propionic acid;
3-(((4-methoxyphenyl)-3-(1-oxisoindolin-yl)propionamide;
3-(((3,4-dimethoxyphenyl)-3-(1-oxisoindolin-2-yl)propionic acid;
3-(((3,4-dimethoxy-phenyl)-3-(1-oxo-1,3-dihydroisoindol-2-yl)propionamide;
3-(((3,4-dimethoxyphenyl)-3-(1-oxisoindolin-2-yl)propionamide;
3-(((3,4-diethoxyphenyl)-3-(1-oxoisoindolin-yl)propionic acid;
methyl 3-(1-oxoisoindolin-2-yl)-3-(3-ethoxy-4-methoxyphenyl)propionate;
3-(((1-oxoisoindolin-2-yl)-3-(3-ethoxy-4-methoxyphenyl)propionic acid;
3-(((1-oxoisoindolin-2-yl)-3-(3-propoxy-4-methoxyphenyl)propionic acid;
3-(((1-oxoisoindolin-2-yl)-3-(3-butoxy-4-methoxyphenyl)propionic acid;
3-(((1-oxoisoindolin-2-yl)-3-(3-propoxy-4-methoxyphenyl)propionamide;
3-(((1-oxoisoindolin-2-yl)-3-(3-butoxy-4-methoxyphenyl)propionamide;
methyl 3-(1-oxoisoindolin-2-yl)-3-(3-butoxy-4-methoxyphenyl)propionate; or
methyl 3-(1-oxoisoindolin-2-yl)-3-(3-propoxy-4-methoxyphenyl)propionate.

Other representative cyclic amides include compounds of the formula: 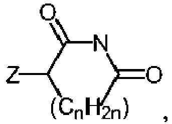 and pharmaceutically acceptable salts, solvates, hydrates, clathrates, stereoisomers, and prodrugs thereof, in which Z is: 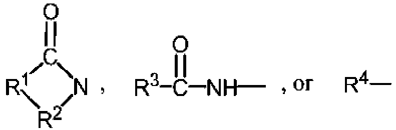 in which:
R¹ is the divalent residue of (i) 3,4-pyridine, (ii) pyrrolidine, (iii) imidizole, (iv) naphthalene, (v) thiophene, or (vi) a straight or branched alkane of 2 to 6 carbon atoms, unsubstituted or substituted with phenyl or phenyl substituted with nitro, cyano, trifluoromethyl, carbethoxy, carbomethoxy, carbopropoxy, acetyl, carbamyl, acctoxy, carboxy, hydroxy, amino, alkyl of 1 to 10 carbon atoms, alkoxy of 1 to 10 carbon atoms, or halo, wherein the divalent bonds of said residue are on vicinal ring carbon atoms;
R² is -CO - or -SO₂ -;
R³ is (i) phenyl substituted with 1 to 3 substituents each selected independently from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (ii) pyridyl, (iii) pyrrolyl, (iv) imidazolyl, (iv) naphthyl, (vi) thienyl, (vii) quinolyl, (viii) furyl, or (ix) indolyl;
R⁴ is alanyl, arginyl, glycyl, phenylglycyl, histidyl, leucyl, isoleucyl, lysyl, methionyl, prolyl, sarcosyl, seryl, homoseryl, threonyl, thyronyl, tyrosyl, valyl, benzimidol-2-yl, benzoxazol-2-yl, phenylsulfonyl, methylphenylsulfonyl, or phenylcarbamoyl; and
n has a value of 1, 2, or 3.

Other representative cyclic amides include compounds of the formula: 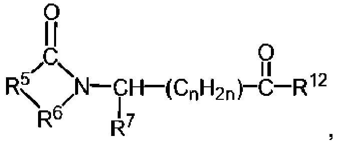 and pharmaceutically acceptable salts, solvates, hydrates, clathrates, stereoisomers, and prodrugs thereof, wherein:
R⁵ is (i) o-phenylene, unsubstituted or substituted with 1 to 4 substituents each selected independently from nitro, cyano, trifluoromethyl, carbethoxy, carbomethoxy, carbopropoxy, acetyl, carbamoyl, acctoxy, carboxy, hydroxy, amino, alkylamino, dialkylamino, acylamino, alkyl of 1 to 10 carbon atoms, alkoxy of 1 to 10 carbon atoms, or halo, or (ii) the divalent residue of pyridine, pyrrolidine, imidizole, naphthalene, or thiophene, wherein the divalent bonds are on vicinal ring carbon atoms;
R⁶ is -CO -, -CH₂-, or -SO₂-;
R⁷ is (i) hydrogen if R⁶ is -SO₂, (ii) straight, branched, or cyclic alkyl of 1 to 12 carbon atoms, (iii) pyridyl, (iv) phenyl or phenyl substituted with one or more substituents each selected independently of the other from nitro, cyano, trifluoromethyl, carbethoxy, carbomethoxy, carbopropoxy, acetyl, carbamoyl, acctoxy, carboxy, hydroxy, amino, alkyl of 1 to 10 carbon atoms, alkoxy of 1 to 10 carbon atoms, or halo, (v) alkyl of 1 to 10 carbon atoms, (vi) benzyl unsubstituted or substituted with 1 to 3 substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (vii) naphthyl, (viii) benzyloxy, or (ix) imidazol-4-yl methyl;
R¹² is -OH, alkoxy of 1 to 12 carbon atoms, or 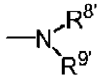
n has a value of 0, 1, 2, or 3;
R^{8'} is hydrogen or alkyl of 1 to 10 carbon atoms; and
R^{9'} is hydrogen, alkyl of 1 to 10 carbon atoms, -COR¹⁰, or -SO₂R¹⁰ in which R¹⁰ is hydrogen, alkyl of 1 to 10 carbon atoms, or phenyl.

Other representative imides include compounds of the formula: 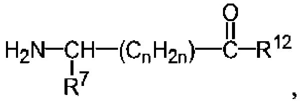 and pharmaceutically acceptable salts, solvates, hydrates, clathrates, stereoisomers, and prodrugs thereof, wherein:
R⁷ is (i) straight, branched, or cyclic alkyl of 1 to 12 carbon atoms, (ii) pyridyl, (iii) phenyl or phenyl substituted with one or more substituents each selected independently of the other from nitro, cyano, trifluoromethyl, carbcthoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, (iv) benzyl unsubstituted or substituted with one to three substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 4 carbon atoms, alkoxy of 1 to 4 carbon atoms, or halo, (v) naphthyl, (vi) benzyloxy, or (vii) imidazol-4-ylmethyl;
R¹² is -OH, alkoxy of 1 to 12 carbon atoms, -O-CH₂-pyridyl, -O-benzyl or 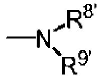 where n has a value of 0, 1, 2, or 3;
R^{8'} is hydrogen or alkyl of 1 to 10 carbon atoms; and
R^{9'} is hydrogen, alkyl of 1 to 10 carbon atoms, -CH₂-pyridyl, benzyl, -COR¹⁰, or - SO₂R¹⁰ in which R¹⁰ is hydrogen, alkyl of 1 to 4 carbon atoms, or phenyl.

Other PDE4 modulators include the imido and amido substituted alkanohydroxamic acids disclosed in WO 99/06041 and U.S. patent no. 6,214,857, each of which is incorporated herein by reference. Examples of such compounds include, but are not limited to: 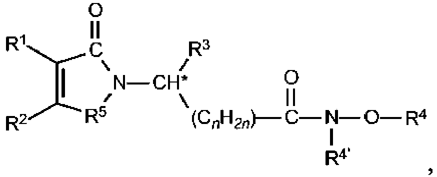 and pharmaceutically acceptable salts, solvates, hydrates, clathrates, stereoisomers, and prodrugs thereof, wherein:
each of R¹ and R², when taken independently of each other, is hydrogen, lower alkyl, or R¹ and R², when taken together with the depicted carbon atoms to which each is bound, is o-phenylene, o-naphthylene, or cyclohexene-1,2-diyl, unsubstituted or substituted with 1 to 4 substituents each selected independently from the group consisting of nitro, cyano, trifluoromethyl, carbethoxy, carbomcthoxy, carbopropoxy, acetyl, carbamoyl, acetoxy, carboxy, hydroxy, amino, alkylamino, dialkylamino, acylamino, alkyl of 1 to 10 carbon atoms, alkoxy of 1 to 10 carbon atoms, and halo;
R³ is phenyl substituted with from one to four substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, alkylthio of 1 to 10 carbon atoms, benzyloxy, cycloalkoxy of 3 to 6 carbon atoms, C₄-C₆-cycloalkylidenemethyl, C₃-C₁₀-alkylidenemethyl, indanyloxy, and halo;
R⁴ is hydrogen, alkyl of 1 to 6 carbon atoms, phenyl, or benzyl;
R^{4'} is hydrogen or alkyl of 1 to 6 carbon atoms;
R⁵ is -CH₂-, -CH₂-CO-, -SO₂-, -S-, or -NHCO-; and
n has a value of 0, 1, or 2.

In some embodiments, the PDE4 modulator is the following compound, or a pharmaceutically acceptable salt, solvate, hydrate, clathrate, stereoisomer, or prodrug thereof:
3-(((3-ethoxy-4-methoxyphenyl)-N-hydroxy-3-(1-oxoisoindolinyl)propionamide;
3-(((3-ethoxy-4-methoxyphenyl)-N-methoxy-3-(1-oxoisoindolinyl)propionamide;
N-benzyloxy-3-(3-ethoxy-4-methoxyphenyl)-3-phthalimidopropionamide;
N-benzyloxy-3-(3-ethoxy-4-methoxyphenyl)-3-(3-nitrophthalimido)propionamide;
N-benzyloxy-3-(3-ethoxy-4-methoxyphenyl)-3-(1-oxoisoindolinyl)propionamide;
3-(((3-ethoxy-4-methoxyphenyl)-N-hydroxy-3-phthalimidopropionamide;
N-hydroxy-3-(3,4-dimethoxyphenyl)-3-phthalimidopropionamide;
3-(((3-ethoxy-4-methoxyphenyl)-N-hydroxy-3-(3-nitrophthalimido)propionamide;
N-hydroxy-3-(3,4-dimethoxyphenyl)-3-(1-oxoisoindolinyl)propionamide;
3-(((3-ethoxy-4-methoxyphenyl)-N-hydroxy-3-(4-methyl-phthalimido)propionamide;
3-(((3-cyclopentyloxy-4-methoxyphenyl)-N-hydroxy-3-phthalimidopropionamide;
3-(((3-ethoxy-4-methoxyphenyl)-N-hydroxy-3-(1,3-dioxo-2,3-dihydro-1H-benzo[f]isoindol-2-yl)propionamide;
N-hydroxy-3-{3-(2-propoxy)-4-methoxyphenyl}-3-phthalimidopropionamide;
3-(((3-ethoxy-4-methoxyphenyl)-3-(3,6-difluorophthalimido)-N-hydroxypropionamide;
3-(((4-aminophthalimido)-3-(3-ethoxy-4-methoxyphenyl)-N-hydroxypropionamide;
3-(((3-aminophthalimido)-3-(3-ethoxy-4-methoxyphenyl)-N-hydroxypropionamide;
3-((3-acetoamidophthalimido)-3-(3-ethoxy-4-methoxyphenyl)-N-hydroxypropionamide;
N-hydroxy-3-(3,4-dimethoxyphenyl)-3-(1-oxoisoindolinyl)propionamide;
3-(((3-cyclopentyloxy-4-methoxyphenyl)-N-hydroxy-3-(1-oxoisoindolinyl) propionamidc; or
N-benzyloxy-3-(3-ethoxy-4-methoxyphenyl)-3-(3-nitrophthalimido)propionamide.

Other PDE4 modulators include the substituted phenethylsulfones substituted on the phenyl group with a oxoisoindine group. Examples of such compounds include, but are not limited to, those disclosed in U.S. patent no. 6,020,358, which is incorporated herein by reference, which include the following: 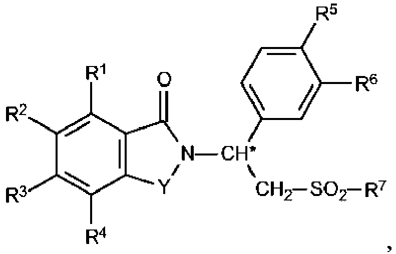 and pharmaceutically acceptable salts, solvates, hydrates, clathrates, stereoisomers, and prodrugs thereof, wherein:
the carbon atom designated * constitutes a center of chirality;
Y is C=O, CH₂, SO₂, or CH₂C=O; each of R¹, R², R³, and R⁴, independently of the others, is hydrogen, halo, alkyl of 1 to 4 carbon atoms, alkoxy of 1 to 4 carbon atoms, nitro, cyano, hydroxy, or -NR⁸R⁹; or any two of R¹, R², R³, and R⁴ on adjacent carbon atoms, together with the depicted phenylene ring are naphthylidene;
each of R⁵ and R⁶, independently of the other, is hydrogen, alkyl of 1 to 4 carbon atoms, alkoxy of 1 to 4 carbon atoms, cyano, or cycloalkoxy of up to 18 carbon atoms;
R⁷ is hydroxy, alkyl of 1 to 8 carbon atoms, phenyl, benzyl, or NR^{8'}R^{9'};
each of R⁸ and R⁹ taken independently of the other is hydrogen, alkyl of 1 to 8 carbon atoms, phenyl, or benzyl, or one of R⁸ and R⁹ is hydrogen and the other is -COR¹⁰ or -SO₂R¹⁰, or R⁸ and R⁹ taken together are tetramethylene, pentamethylene, hexamethylene, or -CH₂CH₂X¹CH₂CH₂- in which X¹ is -O-, -S- or -NH-; and
each of R^{8'} and R^{9'} taken independently of the other is hydrogen, alkyl of 1 to 8 carbon atoms, phenyl, or benzyl, or one of R^{8'} and R^{9'} is hydrogen and the other is COR^{10'} or -SO₂R^{10'}, or R^{8'} and R^{9'} taken together are tetramethylene, pentamethylene, hexamethylene, or -CHCH₂X²CH₂CH₂- in which X² is -O-, -S-, or -NH-.

It will be appreciated that while for convenience the above compounds are identified as phenethylsulfones, they include sulfonamides when R⁷ is NR^{8'}R^{9'}.

In some embodiments, the compounds are those in which Y is C=O or CH₂.

In other embodiments, the compounds are those in which each of R¹, R², R³, and R⁴ independently of the others, is hydrogen, halo, methyl, ethyl, methoxy, ethoxy, nitro, cyano, hydroxy, or -NR⁸R⁹ in which each of R⁸ and R⁹ taken independently of the other is hydrogen or methyl or one of R⁸ and R⁹ is hydrogen and the other is -COCH₃.

In other embodiments, the compounds are those in which one of R¹, R², R³, and R⁴ is -NH₂ and the remaining of R¹, R², R³, and R⁴ arc hydrogen.

In other embodiments, the compounds are those in which one of R¹, R², R³, and R⁴ is -NHCOCH₃ and the remaining of R¹, R², R³, and R⁴ arc hydrogen.

In other embodiments, the compounds are those in which one of R¹, R², R³, and R⁴ is -N(CH₃)₂ and the remaining of R¹, R², R³, and R⁴ are hydrogen.

In other embodiments, the compounds are those in which one of R¹, R², R³, and R⁴ is methyl and the remaining of R¹, R², R³, and R⁴ are hydrogen.

In other embodiments, the compounds are those in which one of R¹, R², R³, and R⁴ is fluoro and the remaining of R¹, R², R³, and R⁴ are hydrogen.

In other embodiments, the compounds are those in which each of R⁵ and R⁶, independently of the other, is hydrogen, methyl, ethyl, propyl, methoxy, ethoxy, propoxy, cyclopentoxy, or cyclohexoxy.

In other embodiments, the compounds are those in which R⁵ is methoxy and R⁶ is monocycloalkoxy, polycycloalkoxy, and benzocycloalkoxy.

In other embodiments, the compounds are those in which R⁵ is methoxy and R⁶ is ethoxy.

In other embodiments, the compounds are those in which R⁷ is hydroxy, methyl, ethyl, phenyl, benzyl, or NR^{8'}R^{9'} in which each of R⁸ and R^{9'} taken independently of the other is hydrogen or methyl.

In other embodiments, the compounds are those in which R⁷ is methyl, ethyl, phenyl, benzyl or NR^{8'}R^{9'} in which each of R^{8'} and R^{9'} taken independently of the other is hydrogen or methyl.

In other embodiments, the compounds are those in which R⁷ is methyl.

In other embodiments, the compounds arc those in which R⁷ is NR^{8'}R^{9'} in which each of R^{8'} and R^{9'} taken independently of the other is hydrogen or methyl.

Other PDE4 modulators include fluoroalkoxy-substituted 1,3-dihydro-isoindolyl compounds disclosed in U.S. patent no. 7,173,058, which is incorporated herein by reference. Representative compounds include those of formula: 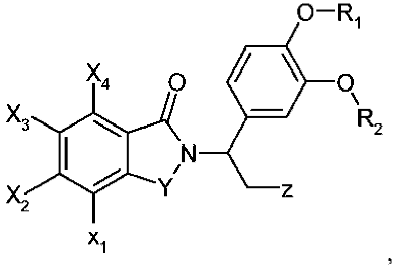 and pharmaceutically acceptable salts, solvates, hydrates, clathrates, stereoisomers, and prodrugs thereof, wherein:
Y is -C(O)-, -CH₂, -CH₂C(O)-, -C(O)CH₂-, or SO₂;
Z is -H, -C(O)R³, -(C₀₋₁-alkyl)-SO₂-(C₁₋₄-alkyl), -C₁₋₈-alkyl, -CH₂OH, CH₂(O)(C₁₋₈-alkyl) or -CN;
R₁ and R² are each independently -CHF₂, -C₁₋₈-alkyl, -C₃₋₁₃-cycloalkyl, or -(C₁₋₁₀-alkyl)(C₃₋₁₀-cycloalkyl), and at least one of R₁ and R² is CHF₂;
R³ is -NR⁴R⁵, -alkyl, -OH, -O-alkyl, phenyl, benzyl, substituted phenyl, or substituted benzyl;
R⁴ and R⁵ are each independently -H, -C₁₋₈-alkyl, -OH, -OC(O)R⁶;
R⁶ is -C₁₋₈-alkyl, -amino(C₁₋₈-alkyl), -phenyl, -benzyl, or -aryl;
X₁ X₂, X₃, and X₄ are each independently -H, -halogen, -nitro, -NH₂, -CF₃, -C₁₋₆-alkyl, -(C₀₋₄-alkyl)-(C₃₋₆-cycloalkyl), (C₀₋₄-alkyl)-NR⁷R⁸_{,} (C₀₋₄-alkyl)-N(H)C(O)-(R⁸), (C₀₋₄-alkyl)-N(H)C(O)N(R⁷R⁸), (C₀₋₄-alkyl)-N(H)C(O)O(R⁷R⁸), (C₀₋₄-alkyl)-OR⁸, (C₀₋₄-alkyl)-imidazolyl, (C₀₋₄-alkyl)-pyrrolyl, (C₀₋₄-alkyl)-oxadiazolyl, or (C₀₋₄-alkyl)-triazolyl, or two of X₁, X₂, X₃, and X₄ may be joined together to form a cycloalkyl or heterocycloalkyl ring, *(e.g.,* X₁ and X₂, X₂ and X₃, X₃ and X₄, X₁ and X₃, X₂ and X₄, or X₁ and X₄ may form a 3, 4, 5, 6, or 7 membered ring which may be aromatic, thereby forming a bicyclic system with the isoindolyl ring); and
R⁷ and R⁸ are each independently H, C₁₋₉-alkyl, C₃₋₆-cycloalkyl, (C₁₋₆-alkyl)-(C₃₋₆-cycloalkyl), (C₁₋₆-alkyl)-N(R⁷R⁸), (C₁₋₆-alkyl)-OR⁸, phenyl, benzyl, or aryl.

Other PDE4 modulators include the enantiomerically pure compounds disclosed in U.S. patent no. 6,962,940; international patent publication nos. WO 2003/080048 and WO 2003/080049; U.S. patent no. 7,312,241 to G. Muller et al.*;* and U.S. patent publication no. 2004/0167199A1, published August 26, 2004, all of which are incorporated herein by reference. In certain embodiments, the compounds are an enantiomer of 2-[1-{3-ethoxy-4-methoxyphenyl)-2-methylsulfonylethyl]-4-acetylaminoisoindoline-1,3-dione and an enantiomer of 3-(3,4-dimethoxy-phenyl)-3-(1-oxo-1,3-dihydro-isoindol-2-yl)-propionamide.

In certain embodiments, the PDE4 modulators provided herein are 3-(3,4-dimethoxy-phenyl)-3-(1-oxo-1,3-dihydro-isoindol-2-yl)-propionamide and cyclopropanecarboxylic acid {2-[1-(3-ethoxy-4-methoxy-phenyl)-2-methanesulfonyl-ethyl]-3-oxo-2,3-dihydro-1H-isoindol-4-yl}-amide, which are available from Celgene Corp., Warren, NJ. 3-(3,4-Dimethoxy-phenyl)-3-(1-oxo-1,3-dihydro-isoindol-2-yl)-propionamidc has the following chemical structure:

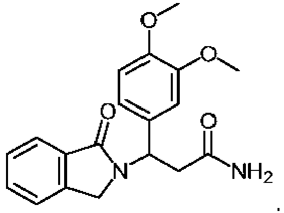

Other PDE4 modulators include, but are not limited to, the cycloalkyl amides and cycloalkyl nitriles of U.S. patent nos. 5,728,844, 5,728,845, 5,968,945, 6,180,644 and 6,518,281, and WO 97/08143 and WO 97/23457, each of which is incorporated herein by reference. Representative compounds include those of formula: 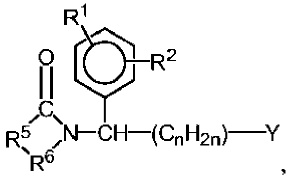 and pharmaceutically acceptable salts, solvates, hydrates, clathrates, stereoisomers, and prodrugs thereof, wherein:
one of R¹ and R² is R³-X- and the other is hydrogen, nitro, cyano, trifluoromethyl, carbo(lower)alkoxy, acetyl, carbamoyl, acetoxy, carboxy, hydroxy, amino, lower alkyl, lower alkoxy, halo, or R³-X-;
R³ is monocycloalkyl, bicycloalkyl, or benzocycloalkyl of up to 18 carbon atoms;
X is a carbon-carbon bond, -CH₂-, or -O-;
R⁵ is (i) o-phenylene, unsubstituted or substituted with 1 to 3 substitucnts each selected independently from nitro, cyano, halo, trifluoromethyl, carbo(lower)alkoxy, acetyl, or carbamoyl, unsubstituted or substituted with lower alkyl, acetoxy, carboxy, hydroxy, amino, lower alkylamino, lower acylamino, or lower alkoxy; (ii) a vicinally divalent residue of pyridine, pyrrolidine, imidazole, naphthalene, or thiophene, wherein the divalent bonds arc on vicinal ring carbon atoms; (iii) a vicinally divalent cycloalkyl or cycloalkenyl of 4-10 carbon atoms, unsubstituted or substituted with 1 to 3 substituents each selected independently from the group consisting of nitro, cyano, halo, trifluoromethyl, carbo(lower)alkoxy, acetyl, carbamoyl, acetoxy, carboxy, hydroxy, amino, lower alkylamino, lower alkyl, lower alkoxy, or phenyl; (iv) vinylene di-substituted with lower alkyl; or (v) ethylene, unsubstituted or monosubstituted or disubstituted with lower alkyl;
R⁶ is -CO-, -CH₂-, or -CH₂CO-;
Y is -COZ, -C≡N, -OR⁸, lower alkyl, or aryl;
Z is NH₂, -OH, -NHR, -R⁹, or -OR⁹
R⁸ is hydrogen or lower alkyl;
R⁹ is lower alkyl or benzyl; and
n has a value of 0, 1, 2, or 3.
In one embodiment, one of R¹ and R² is R³-X- and the other is hydrogen, nitro, cyano, trifluoromethyl, carbo(lower)alkoxy, acetyl, carbamoyl, acetoxy, carboxy, hydroxy, amino, lower alkyl, lower alkoxy, halo, or R³-X-;
R³ is monocycloalkyl of up to 10 carbon atoms, polycycloalkyl of up to 10 carbon atoms, or benzocyclic alkyl of up to 10 carbon atoms;
X is -CH₂-, or -O-;
R⁵ is (i) the vicinally divalent residue of pyridine, pyrrolidine, imidazole, naphthalene, or thiophene, wherein the two bonds of the divalent residue are on vicinal ring carbon atoms;
(ii) a vicinally divalent cycloalkyl of 4-10 carbon atoms, unsubstituted or substituted with 1 to 3 substituents each selected independently from the group consisting of nitro, cyano, halo, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or phenyl;
(iii) di-substituted vinylene, substituted with nitro, cyano, trifluoromethyl, carbethoxy, carbomethoxy, carbopropoxy, acetyl, carbamoyl, carbamoyl substituted with and alkyl of 1 to 3 carbon atoms, acctoxy, carboxy, hydroxy, amino, amino substituted with an alkyl of 1 to 3 carbon atoms, alkyl of 1 to 4 carbon atoms, alkoxy of 1 to 4 carbon atoms, or halo;
(iv) ethylene, unsubstituted or substituted with 1 to 2 substituents each selected independently from nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo;
R⁶ is -CO-, -CH₂-, or -CH₂CO-;
Y is -COX, -C≡N, -OR⁸, alkyl of 1 to 5 carbon atoms, or aryl;
X is -NH₂, -OH, -NHR, -R⁹, -OR⁹, or alkyl of 1 to 5 carbon atoms;
R⁸ is hydrogen or lower alkyl;
R⁹ is alkyl or benzyl; and,
n has a value of 0, 1, 2, or 3.
In another embodiment, one of R¹ and R² is R³-X- and the other is hydrogen, nitro, cyano, trifluoromethyl, carbo(lower)alkoxy, acetyl, carbamoyl, acetoxy, carboxy, hydroxy, amino, lower alkyl, lower alkoxy, halo, HF₂CO, F₃CO, or R³-X-;
R³ is monocycloalkyl, bicycloalkyl, benzocyclo alkyl of up to 18 carbon atoms, tetrahydropyran, or tetrahydrofuran;
X is a carbon-carbon bond, -CH₂-, -O-, or -N=;
R⁵ is (i) o-phenylene, unsubstituted or substituted with 1 to 3 substituents each selected independently from nitro, cyano, halo, trifluoromethyl, carbo(lower)alkoxy, acetyl, or carbamoyl, unsubstituted or substituted with lower alkyl, acetoxy, carboxy, hydroxy, amino, lower alkylamino, lower acylamino, or lower alkoxy; (ii) a vicinally divalent residue of pyridine, pyrrolidine, imidazole, naphthalene, or thiophene, wherein the divalent bonds are on vicinal ring carbon atoms; (iii) a vicinally divalent cycloalkyl or cycloalkenyl of 4-10 carbon atoms, unsubstituted or substituted with 1 or more substituents each selected independently from the group consisting of nitro, cyano, halo, trifluoromethyl, carbo(lower)alkoxy, acetyl, carbamoyl, acctoxy, carboxy, hydroxy, amino, lower alkylamino, lower alkyl, lower alkoxy, or phenyl; (iv) vinylene di-substitutcd with lower alkyl; or (v) ethylene, unsubstituted or monosubstituted or disubstituted with lower alkyl;
R⁶ is -CO-, -CH₂-, or -CH₂CO-;
Y is -COX, -C≡N, -OR⁸, alkyl of 1 to 5 carbon atoms, or aryl;
X is -NH₂, -OH, -NHR, -R⁹, -OR⁹, or alkyl of 1 to 5 carbon atoms;
R⁸ is hydrogen or lower alkyl;
R⁹ is alkyl or benzyl; and,
n has a value of 0, 1, 2, or 3.

Other representative compounds include those of formula: 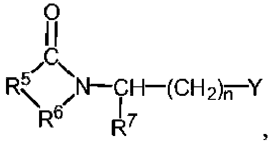 and pharmaceutically acceptable salts, solvates, hydrates, clathrates, stereoisomers, and prodrugs thereof, wherein:
Y is -C≡N or CO(CH₂)ₘCH₃;
m is 0, 1, 2, or 3;
R⁵ is (i) o-phenylene, unsubstituted or substituted with 1 to 3 substituents each selected independently from nitro, cyano, trifluoromethyl, carbcthoxy, carbomethoxy, carbopropoxy, acetyl, carbamoyl, carbamoyl substituted with and alkyl of 1 to 3 carbon atoms, acctoxy, carboxy, hydroxy, amino, amino substituted with an alkyl of 1 to 3 carbon atoms, alkyl of 1 to 4 carbon atoms, alkoxy of 1 to 4 carbon atoms, or halo; (ii) the divalent residue of pyridine, pyrrolidine, imidizole, naphthalene, or thiophene, wherein the divalent bonds are on vicinal ring carbon atoms; (iii) a divalent cycloalkyl of 4-10 carbon atoms, unsubstituted or substituted with one or more substituents each selected independently of the other from the group consisting of nitro, cyano, trifluoromethyl, carbcthoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, phenyl or halo; (iv) di-substituted vinylene, substituted with nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo; or (v) ethylene, unsubstituted or substituted with 1 to 2 substituents each selected independently from nitro, cyano, trifluoromethyl, carbethoxy, carbomethoxy, carbopropoxy, acetyl, carbamoyl, carbamoyl substituted with and alkyl of 1 to 3 carbon atoms, acctoxy, carboxy, hydroxy, amino, amino substituted with an alkyl of 1 to 3 carbon atoms, alkyl of 1 to 4 carbon atoms, alkoxy of 1 to 4 carbon atoms, or halo;
R⁶ is -CO-, -CH₂-, -CH₂CO-, or -SO₂-;
R⁷ is (i) straight or branched alkyl of 1 to 12 carbon atoms; (ii) cyclic or bicyclic alkyl of 1 to 12 carbon atoms; (iii) pyridyl; (iv) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, straight, branched, cyclic, or bicyclic alkyl of 1 to 10 carbon atoms, straight, branched, cyclic, or bicyclic alkoxy of 1 to 10 carbon atoms, CH₂R where R is a cyclic or bicyclic alkyl of 1 to 10 carbon atoms, or halo; (v) benzyl substituted with one to three substituents each selected independently from the group consisting of nitro, cyano, trifluoromethyl, carbethoxy, carbomethoxy, carbopropoxy, acetyl, carbamoyl, acctoxy, carboxy, hydroxy, amino, alkyl of 1 to 4 carbon atoms, alkoxy of 1 to 10 carbon atoms, or halo; (vi) naphthyl; or (vii) benzyloxy; and
n has a value of 0, 1, 2, or 3.

In another embodiment, the PDE4 modulators include those of formula: 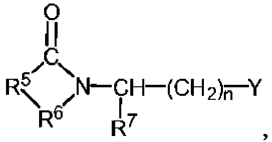 and pharmaceutically acceptable salts, solvates, hydrates, clathrates, stereoisomers, and prodrugs thereof, wherein:
R⁵ is (i) the divalent residue of pyridine, pyrrolidine, imidizole, naphthalene, or thiophene, wherein the divalent bonds are on vicinal ring carbon atoms; (ii) a divalent cycloalkyl of 4-10 carbon atoms, unsubstituted or substituted with one or more substituents each selected independently of the other from the group consisting of nitro, cyano, trifluoromethyl, carbethoxy, carbomethoxy, carbopropoxy, acetyl, carbamoyl, acctoxy, carboxy, hydroxy, amino, substituted amino, alkyl of 1 to 10 carbon atoms, alkoxy of 1 to 10 carbon atoms, phenyl or halo; (iii) di-substituted vinylene, substituted with nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo; or (iv) ethylene, unsubstituted or substituted with 1 to 2 substituents each selected independently from nitro, cyano, trifluoromethyl, carbethoxy, carbomethoxy, carbopropoxy, acetyl, carbamoyl, carbamoyl substituted with and alkyl of 1 to 3 carbon atoms, acetoxy, carboxy, hydroxy, amino, amino substituted with an alkyl of 1 to 3 carbon atoms, alkyl of 1 to 4 carbon atoms, alkoxy of 1 to 4 carbon atoms, or halo;
R⁶ is -CO-, CH₂-, CH₂CO-, or -SO₂-;
R⁷ is (i) cyclic or bicyclic alkyl of 4 to 12 carbon atoms; (ii) pyridyl; (iii) phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, straight, branched, cyclic, or bicyclic alkyl of 1 to 10 carbon atoms, straight, branched, cyclic, or bicyclic alkoxy of 1 to 10 carbon atoms, CH₂R where R is a cyclic or bicyclic alkyl of 1 to 10 carbon atoms, or halo; (iv) benzyl substituted with one to three substituents each selected independently from the group consisting of nitro, cyano, trifluoromethyl, carbcthoxy, carbomethoxy, carbopropoxy, acetyl, carbamoyl, acetoxy, carboxy, hydroxy, amino, alkyl of 1 to 4 carbon atoms, alkoxy of 1 to 10 carbon atoms, or halo; (v) naphthyl; or (vi) benzyloxy; and
Y is COX, -C≡N, OR⁸, alkyl of 1 to 5 carbon atoms, or aryl;
X is -NH₂, -OH, -NHR, -R⁹, -OR⁹, or alkyl of 1 to 5 carbon atoms;
R⁸ is hydrogen or lower alkyl;
R⁹ is alkyl or benzyl; and
n has a value of 0, 1, 2, or 3.

Other PDE4 modulators include, but are not limited to, the aryl amides (for example, an embodiment being N-benzoyl-3-amino-3-(3',4'-dimethoxyphenyl)-propanamide) of U.S. patent nos. 5,801,195, 5,736,570, 6,046,221 and 6,284,780, each of which is incorporated herein by reference. Representative compounds include those of formula: 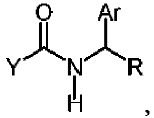 and pharmaceutically acceptable salts, solvates, hydrates, clathrates, stereoisomers, and prodrugs thereof, wherein:
Ar is (i) straight, branched, or cyclic, unsubstituted alkyl of 1 to 12 carbon atoms; (ii) straight, branched, or cyclic, substituted alkyl of 1 to 12 carbon atoms; (iii) phenyl; (iv) phenyl substituted with one or more substituents each selected independently of the other from the group consisting of nitro, cyano, trifluoromethyl, carbethoxy, carbomethoxy, carbopropoxy, acetyl, carbamoyl, acctoxy, carboxy, hydroxy, amino, substituted amino, alkyl of 1 to 10 carbon atoms, alkoxy of 1 to 10 carbon atoms, or halo; (v) heterocycle; or (vi) heterocycle substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo;
R is -H, alkyl of 1 to 10 carbon atoms, CH₂OH, CH₂CH₂OH, or CH₂COZ where Z is alkoxy of 1 to 10 carbon atoms, benzyloxy, or NHR¹ where R¹ is H or alkyl of 1 to 10 carbon atoms; and
Y is i) a phenyl or heterocyclic ring, unsubstituted or substituted one or more substituents each selected independently one from the other from nitro, cyano, trifluoromethyl, carbethoxy, carbomethoxy, carbopropoxy, acetyl, carbamoyl, acctoxy, carboxy, hydroxy, amino, alkyl of 1 to 10 carbon atoms, alkoxy of 1 to 10 carbon atoms, or halo or ii) naphthyl.

Other examples of the compounds include those of formula: 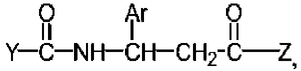 and pharmaceutically acceptable salts, solvates, hydrates, clathrates, stereoisomers, and prodrugs thereof, wherein:
Ar is 3,4-disubstituted phenyl where each substituent is selected independently of the other from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo;
Z is alkoxy of 1 to 10 carbon atoms, benzyloxy, amino, or alkylamino of 1 to 10 carbon atoms; and
Y is (i) a phenyl, unsubstituted or substituted with one or more substituents each selected, independently one from the other, from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, or (ii) naphthyl.

Other PDE4 modulators include, but are not limited to, the imide/amide ethers and alcohols (for example, 3-phthalimido-3-(3',4'-dimethoxyphenyl) propan-1-ol) disclosed in U.S. patent no. 5,703,098, which is incorporated herein by reference. Examples include compounds the formula: 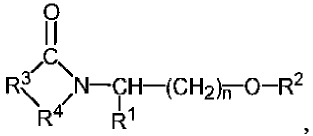 and pharmaceutically acceptable salts, solvates, hydrates, clathrates, stereoisomers, and prodrugs thereof, wherein:
R¹ is (i) straight, branched, or cyclic, unsubstituted alkyl of 1 to 12 carbon atoms; (ii) straight, branched, or cyclic, substituted alkyl of 1 to 12 carbon atoms; (iii) phenyl; or (iv) phenyl substituted with one or more substituents each selected independently of the other from the group consisting of nitro, cyano, trifluoromethyl, carbethoxy, carbomcthoxy, carbopropoxy, acetyl, carbamoyl, acctoxy, carboxy, hydroxy, amino, acylamino, alkylamino, di(alkyl) amino, alkyl of 1 to 10 carbon atoms, cycloalkyl of 3 to 10 carbon atoms, bicycloalkyl of 5 to 12 carbon atoms, alkoxy of 1 to 10 carbon atoms, cycloalkoxy of 3 to 10 carbon atoms, bicycloalkoxy of 5 to 12 carbon atoms, and halo;
R² is hydrogen, alkyl of 1 to 8 carbon atoms, benzyl, pyridylmethyl, or alkoxymethyl;
R³ is (i) ethylene, (ii) vinylene, (iii) a branched alkylene of 3 to 10 carbon atoms, (iv) a branched alkenylene of 3 to 10 carbon atoms, (v) cycloalkylene of 4 to 9 carbon atoms unsubstituted or substituted with one or more substituents each selected independently from the group consisting of nitro, cyano, trifluoromethyl, carbethoxy, carbomethoxy, carbopropoxy, acetyl, carbamoyl, acetoxy, carboxy, hydroxy, amino, amino substituted with alkyl of 1 to 6 carbon atoms, amino substituted with acyl of 1 to 6 carbon atoms, alkyl of 1 to 10 carbon atoms, alkoxy of 1 to 12 carbon atoms, and halo, (vi) cycloalkenylene of 4 to 9 carbon atoms unsubstituted or substituted with one or more substituents each selected independently from the group consisting of nitro, cyano, trifluoromethyl, carbethoxy, carbomethoxy, carbopropoxy, acetyl, carbamoyl, acctoxy, carboxy, hydroxy, amino, amino substituted with alkyl of 1 to 6 carbon atoms, amino substituted with acyl of 1 to 6 carbon atoms, alkyl of 1 to 10 carbon atoms, alkoxy of 1 to 12 carbon atoms, and halo, (vii) o-phenylene unsubstituted or substituted with one or more substituents each selected independently from the group consisting of nitro, cyano, trifluoromethyl, carbethoxy, carbomethoxy, carbopropoxy, acetyl, carbamoyl, acetoxy, carboxy, hydroxy, amino, amino substituted with alkyl of 1 to 6 carbon atoms, amino substituted with acyl of 1 to 6 carbon atoms, alkyl of 1 to 10 carbon atoms, alkoxy of 1 to 12 carbon atoms, and halo, (viii) naphthyl, or (ix) pyridyl;
R⁴ is -CX-, -CH₂- or -CH₂CX-;
X is O or S; and
n is 0, 1, 2, or 3.

Other PDE4 modulators include, but are not limited to, the succinimides and maleimides (for example methyl 3-(3',4',5'6'-petrahydrophthalimdo)-3-(3",4"-dimethoxyphenyl)propionate) disclosed in U.S. patent no. 5,658,940, which is incorporated herein by reference. Examples include compounds of formula: 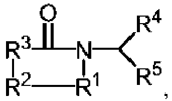 and pharmaceutically acceptable salts, solvates, hydrates, clathrates, stereoisomers, and prodrugs thereof, wherein:
R¹ is -CH₂-, CH₂CO-, or -CO-;
R² and R³ taken together are (i) ethylene unsubstituted or substituted with alkyl of 1-10 carbon atoms or phenyl, (ii) vinylene substituted with two substituents each selected, independently of the other, from the group consisting of alkyl of 1-10 carbon atoms and phenyl, or (iii) a divalent cycloalkyl of 5-10 carbon atoms, unsubstituted or substituted with one or more substituents each selected independently of the other from the group consisting of nitro, cyano, trifluoromethyl, carbethoxy, carbomethoxy, carbopropoxy, acetyl, carbamoyl unsubstituted or substituted with alkyl of 1-3 carbon atoms, acetoxy, carboxy, hydroxy, amino, substituted amino, alkyl of 1 to 10 carbon atoms, alkoxy of 1 to 10 carbon atoms, norbornyl, phenyl or halo;
R⁴ is (i) straight or branched unsubstituted alkyl of 4 to 8 carbon atoms, (ii) cycloalkyl or bicycloalkyl of 5-10 carbon atoms, unsubstituted or substituted with one or more substituents each selected independently of the other from the group consisting of nitro, cyano, trifluoromethyl, carbethoxy, carbomethoxy, carbopropoxy, acetyl, carbamoyl, acctoxy, carboxy, hydroxy, amino, substituted amino, branched, straight or cyclic alkyl of 1 to 10 carbon atoms, alkoxy of 1 to 10 carbon atoms, phenyl or halo, (iii) phenyl substituted with one or more substituents each selected independently of the other from the group consisting of nitro, cyano, trifluoromethyl, carbethoxy, carbomethoxy, carbopropoxy, acetyl, carbamoyl, acctoxy, carboxy, hydroxy, amino, substituted amino, alkyl of 1 to 10 carbon atoms, alkoxy of 1 to 10 carbon atoms, cycloalkyl or bicyctoalkyl of 3 to 10 carbon atoms, cycloalkoxy or bicycloalkoxy of 3 to 10 carbon atoms, phenyl or halo, (iv) pyridine or pyrrolidine, unsubstituted or substituted with one or more substituents each selected independently of the other from the group consisting of nitro, cyano, trifluoromethyl, carbcthoxy, carbomethoxy, carbopropoxy, acetyl, carbamoyl, acctoxy, carboxy, hydroxy, amino, substituted amino, alkyl of 1 to 10 carbon atoms, alkoxy of 1 to 10 carbon atoms, phenyl or halo; and,
R⁵ is -COX, -CN, -CH₂COX, alkyl of 1 to 5 carbon atoms, aryl, -CH₂OR, -CH₂ aryl, or -CH₂OH,
where X is NH₂, OH, NHR, or OR⁶,
where R is lower alkyl; and
where R⁶ is alkyl or benzyl.

Other PDE4 modulators include, but are not limited to, substituted imides (for example, 2-phthalimido-3-(3',4'-dimethoxyphenyl) propane) disclosed in U.S. patent no. 6,429,221, which is incorporated herein by reference. Examples include compounds of the formula: 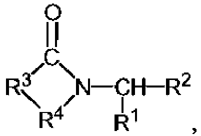 and pharmaceutically acceptable salts, solvates, hydrates, clathrates, stereoisomers, and prodrugs thereof, wherein:
R¹ is (i) straight, branched, or cyclic alkyl of 1 to 12 carbon atoms, (ii) phenyl or phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, straight or branched alkyl of 1 to 10 carbon atoms, alkoxy of 1 to 10 carbon atoms, or halo, (iii) benzyl or benzyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo, or (iv) -Y-Ph where Y is a straight, branched, or cyclic alkyl of 1 to 12 carbon atoms and Ph is phenyl or phenyl substituted with one or more substituents each selected independently of the other from nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, or halo;
R² is -H, a branched or unbranched alkyl of 1 to 10 carbon atoms, phenyl, pyridyl, heterocycle, -CH₂-aryl, or -CH₂-heterocycle;
R³ is i) ethylene, ii) vinylene, iii) a branched alkylene of 3 to 10 carbon atoms, iv) a branched alkenylene of 3 to 10 carbon atoms, v) cycloalkylene of 4 to 9 carbon atoms unsubstituted or substituted with 1 to 2 substituents each selected independently from nitro, cyano, trifluoromethyl, carbethoxy, carbomethoxy, carbopropoxy, acetyl, carbamoyl, acctoxy, carboxy, hydroxy, amino, substituted amino, alkyl of 1 to 4 carbon atoms, alkoxy of 1 to 4 carbon atoms, or halo, vi) cycloalkenylene of 4 to 9 carbon atoms unsubstituted or substituted with 1 to 2 substituents each selected independently from nitro, cyano, trifluoromethyl, carbethoxy, carbomethoxy, carbopropoxy, acetyl, carbamoyl, acetoxy, carboxy, hydroxy, amino, substituted amino, alkyl of 1 to 4 carbon atoms, alkoxy of 1 to 4 carbon atoms, or halo, or vii) o-phenylene unsubstituted or substituted with 1 to 2 substituents each selected independently from nitro, cyano, trifluoromethyl, carbethoxy, carbomethoxy, carbopropoxy, acetyl, carbamoyl, acctoxy, carboxy, hydroxy, amino, substituted amino, alkyl of 1 to 4 carbon atoms, alkoxy 1 to 4 carbon atoms, or halo; and,
R⁴ is -CX, or -CH₂ ;
X is O or S.

Other PDE4 modulators include, but are not limited to, substituted 1,3,4-oxadiazoles (for example, 2-[1-(3-cyclopentyloxy-4-methoxyphenyl)-2-(1,3,4-oxadiazole-2-yl)ethyl]-5-methylisoindoline-1,3-dione) disclosed in U.S. patent no. 6,326,388, which is incorporated herein by reference. Examples include compounds of formula: 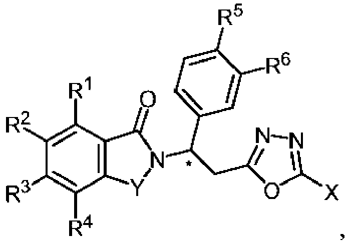 and pharmaceutically acceptable salts, solvates, hydrates, clathrates, stereoisomers, and prodrugs thereof, wherein:
the carbon atom designated* constitutes a center of chirality;
Y is C=O, CH₂, SO₂ or CH₂C=O;
X is hydrogen, or alkyl of 1 to 4 carbon atoms;
each of R¹, R², R³, and R⁴, independently of the others, is hydrogen, halo, trifluoromethyl, acetyl, alkyl of 1 to 8 carbon atoms, alkoxy of 1 to 4 carbon atoms, nitro, cyano, hydroxy, -CH₂NR⁸R⁹, -(CH₂)₂NR⁸R⁹, or -NR⁸R⁹ or
any two of R¹, R², R³, and R⁴ on adjacent carbon atoms, together with the depicted benzene ring are naphthylidene, quinoline, quinoxaline, benzimidazole, benzodioxole or 2-hydroxybenzimidazole;
each of R⁵ and R⁶, independently of the other, is hydrogen, alkyl of 1 to 4 carbon atoms, alkoxy of 1 to 6 carbon atoms, cyano, bcnzocycloalkoxy, cycloalkoxy of up to 18 carbon atoms, bicyloalkoxy of up to 18 carbon atoms, tricylcoalkoxy of up to 18 carbon atoms, or cycloalkylalkoxy of up to 18 carbon atoms;
each of R⁸ and R⁹, taken independently of the other is hydrogen, straight or branched alkyl of 1 to 8 carbon atoms, phenyl, benzyl, pyridyl, pyridylmethyl, or one of R⁸ and R⁹ is hydrogen and the other is -COR¹⁰, or-SO₂R¹⁰, or R⁸ and R⁹ taken together are tetramethylene, pentamethylene, hexamethylene, -CH=NCH=CH-, or -CH₂CH₂X¹CH₂CH₂-in which X¹ is -O- -S-, or -NH-,
R¹⁰ is hydrogen, alkyl of 1 to 8 carbon atoms, cycloalkyl, cycloalkylmethyl of up to 6 carbon atoms, phenyl, pyridyl, benzyl, imidazolylmethyl, pyridylmethyl, NR¹¹R¹², CH₂R¹⁴R¹⁵, or NR¹¹R¹²,
wherein R¹⁴ and R¹⁵, independently of each other, are hydrogen, methyl, ethyl, or propyl, and
wherein R¹¹ and R¹², independently of each other, are hydrogen, alkyl of 1 to 8 carbon atoms, phenyl, or benzyl.

In certain embodiments, the compounds include those of formula: 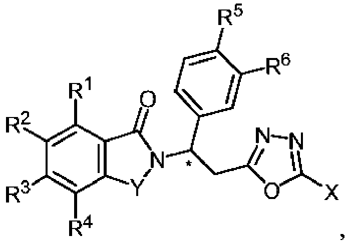 and pharmaceutically acceptable salts, solvates, hydrates, clathrates, stereoisomers, and prodrugs thereof, wherein:
the carbon atom designated* constitutes a center of chirality;
Y is C=O, CH₂, SO₂ or CH₂C=O;
X is hydrogen, or alkyl of 1 to 4 carbon atoms;
   (i) each of R¹, R², R³, and R⁴, independently of the others, is hydrogen, halo, trifluoromethyl, acetyl, alkyl of 1 to 8 carbon atoms, alkoxy of 1 to 4 carbon atoms, nitro, cyano, hydroxy, -CH₂NR⁸R⁹, -(CH₂)₂NR⁸R⁹, or -NR⁸R⁹ or
   (ii) any two of R¹, R², R³, and R⁴ on adjacent carbon atoms, together with the depicted benzene ring to which they arc bound are naphthylidene, quinoline, quinoxaline, benzimidazole, benzodioxole or 2-hydroxybenzimidazole;
each of R⁵ and R⁶, independently of the other, is hydrogen, alkyl of 1 to 4 carbon atoms, alkoxy of 1 to 6 carbon atoms, cyano, benzocycloalkoxy, cycloalkoxy of up to 18 carbon atoms, bicyloalkoxy of up to 18 carbon atoms, tricylcoalkoxy of up to 18 carbon atoms, or cycloalkylalkoxy of up to 18 carbon atoms;
   (i) each of R⁸ and R⁹, independently of the other, is hydrogen, alkyl of 1 to 8 carbon atoms, phenyl, benzyl, pyridyl, pyridylmethyl, or
   (ii) one of R⁸ and R⁹ is hydrogen and the other is -COR¹⁰, or -SO₂R¹⁰, in which R¹⁰ is hydrogen, alkyl of 1 to 8 carbon atoms, cycloalkyl, cycloalkylmethyl of up to 6 carbon atoms, phenyl, pyridyl, benzyl, imidazolylmethyl, pyridylmethyl, NR¹¹R¹², or CH₂NR¹⁴R¹⁵, wherein R¹¹ and R¹², independently of each other, are hydrogen, alkyl of 1 to 8 carbon atoms, phenyl, or benzyl and R¹⁴ and R¹⁵, independently of each other, are hydrogen, methyl, ethyl, or propyl; or
   (iii) R⁸ and R⁹ taken together are tetramethylene, pentamethylene, hexamethylene, -CH=NCH=CH-, or -CH₂CH₂X¹CH₂CH₂- in which X¹ is -O-, -S-, or -NH-.

Other PDE4 modulators include, but are not limited to, cyano and carboxy derivatives of substituted styrenes (for example, 3,3-bis-(3,4-dimethoxyphenyl) acrylonitrile) disclosed in U.S. patent nos. 5,929,117,6,130,226,6,262,10 and 6,479,554, each of which is incorporated herein by reference. Examples include compounds of formula: 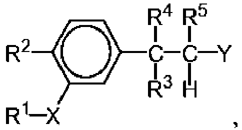 and pharmaceutically acceptable salts, solvates, hydrates, clathrates, stereoisomers, and prodrugs thereof, wherein:
(a) X is -O- or -(CₙH₂ₙ)- in which n has a value of 0, 1, 2, or 3, and R¹ is alkyl of one to 10 carbon atoms, monocycloalkyl of up to 10 carbon atoms, polycycloalkyl of up to 10 carbon atoms, or benzocyclic alkyl of up to 10 carbon atoms, or
(b) X is -CH= and R¹ is alkylidene of up to 10 carbon atoms, monocycloalkylidene of up to 10 carbon atoms, or bicycloalkylidene of up to 10 carbon atoms;
   R² is hydrogen, nitro, cyano, trifluoromethyl, carbethoxy, carbomethoxy, carbopropoxy, acetyl, carbamoyl, acetoxy, carboxy, hydroxy, amino, lower alkyl, lower alkylidenemethyl, lower alkoxy, or halo;
   R³ is (i) phenyl, unsubstituted or substituted with 1 or more substituents each selected independently from nitro, cyano, halo, trifluoromethyl, carbethoxy, carbomethoxy, carbopropoxy, acetyl, carbamoyl, carbamoyl substituted with alkyl of 1 to 3 carbon atoms, acetoxy, carboxy, hydroxy, amino, amino substituted with an alkyl of 1 to 5 carbon atoms, alkyl of up to 10 carbon atoms, cycloalkyl of up to 10 carbon atoms, alkoxy of up to 10 carbon atoms, cycloalkoxy of up to 10 carbon atoms, alkylidenemethyl of up to 10 carbon atoms, cycloalkylidenemethyl of up to 10 carbon atoms, phenyl, or methylenedioxy; (ii) pyridine, substituted pyridine, pyrrolidine, imidizole, naphthalene, or thiophene; (iii) cycloalkyl of 4-10 carbon atoms, unsubstituted or substituted with 1 or more substituents each selected independently from the group consisting of nitro, cyano, halo, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, phenyl;
   each of R⁴ and R⁵ taken individually is hydrogen or R⁴ and R⁵ taken together are a carbon-carbon bond;
   Y is -COZ, -C= N, or lower alkyl of 1 to 5 carbon atoms;
   Z is -OH, -NR⁶R⁶, -R⁷, or -OR⁷; R⁶ is hydrogen or lower alkyl; and R⁷ is alkyl or benzyl.

In some embodiments, ODE4 modulators include compounds of formula: 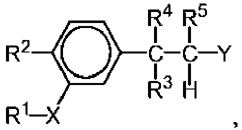 and pharmaceutically acceptable salts, solvates, hydrates, clathrates, stereoisomers, and prodrugs thereof, wherein:
(a) X is -O- or -(CₙH₂ₙ)- in which n has a value of 0, 1, 2, or 3, and R¹ is alkyl of one to 10 carbon atoms, monocycloalkyl of up to 10 carbon atoms, polycycloalkyl of up to 10 carbon atoms, or benzocyclic alkyl of up to 10 carbon atoms, or
(b) X is -CH= and R¹ is alkylidene of up to 10 carbon atoms, monocycloalkylidene of up to 10 carbon atoms, or bicycloalkylidene of up to 10 carbon atoms;
   R² is hydrogen, nitro, cyano, trifluoromethyl, carbethoxy, carbomethoxy, carbopropoxy, acetyl, carbamoyl, acetoxy, carboxy, hydroxy, amino, lower alkyl, lower alkylidenemethyl, lower alkoxy, or halo;
   R³ is pyrrolidine, imidazole or thiophene unsubstituted or substituted with 1 or more substituents each selected independently from the group consisting of nitro, cyano, halo, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or phenyl;
   each of R⁴ and R⁵ taken individually is hydrogen or R⁴ and R⁵ taken together are a carbon-carbon bond;
   Y is -COZ, C≡N, or lower alkyl of 1 to 5 carbon atoms;
   Z is -OH, -NR⁶R⁶, -R⁷, or -OR⁷; R⁶ is hydrogen or lower alkyl; and R⁷ is alkyl or benzyl.

In some embodiments, provided herein are compounds of the formula: 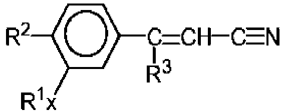 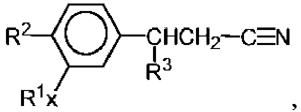 and pharmaceutically acceptable salts, solvates, hydrates, clathrates, stereoisomers, and prodrugs thereof, wherein:
(a) X is -O- or -(CₙH₂ₙ)- in which n has a value of 0, 1, 2, or 3, and R¹ is alkyl of up to 10 carbon atoms, monocycloalkyl of up to 10 carbon atoms, polycycloalkyl of up to 10 carbon atoms, or benzocyclic alkyl of up to 10 carbon atoms, or
(b) X is -CH=, and R¹ is alkylidene of up to 10 carbon atoms or monocycloalkylidene of up to 10 carbon atoms;
   R² is hydrogen, nitro, cyano, trifluoromethyl, carbethoxy, carbomethoxy, carbopropoxy, acetyl, carbamoyl, acetoxy, carboxy, hydroxy, amino, lower alkyl, lower alkoxy, or halo; and
   R³ is (i) phenyl or naphthyl, unsubstituted or substituted with 1 or more substituents each selected independently from nitro, cyano, halo, trifluoromethyl, carbethoxy, carbomethoxy, carbopropoxy, acetyl, carbamoyl, or carbamoyl substituted with alkyl of 1 to 3 carbon atoms, acetoxy, carboxy, hydroxy, amino, amino substituted with an alkyl of 1 to 5 carbon atoms, alkoxy or cycloalkoxy of 1 to 10 carbon atoms; or (ii) cycloalkyl of 4 to 10 carbon atoms, unsubstituted or substituted with one or more substituents each selected independently from the group consisting of nitro, cyano, halo, trifluoromethyl, carbethoxy, carbomethoxy, carbopropoxy, acetyl, carbamoyl, acetoxy, carboxy, hydroxy, amino, substituted amino, alkyl of 1 to 10 carbon atoms, alkoxy of 1 to 10 carbon atoms, or phenyl.

In one embodiment, the compound is of formula:

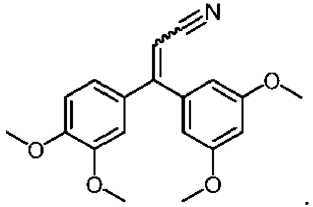

Other PDE4 modulators include, but arc not limited to, isoindoline-1-one and isoindoline-1,3-dione substituted in the 2-position with an α-(3,4-disubstituted phenyl)alkyl 1 group and in the 4- and/or 5-position with a nitrogen-containing group disclosed in WO 01/34606 and U.S. patent no. 6,667,316, which are incorporated herein by reference. Examples include compounds of formula: 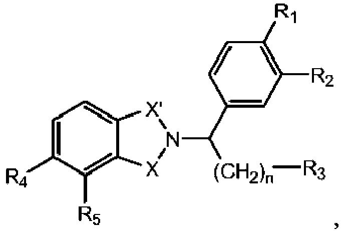 and pharmaceutically acceptable salts, solvates, hydrates, clathrates, stereoisomers, and prodrugs thereof, wherein:
one of X and X' is =C=O or =SO₂, and the other of X and X' is =C=O, =CH₂, =SO₂ or =CH₂C=O;
n is 1, 2 or 3;
R₁ and R₂ are each independently (C₁-C₄)alkyl, (C₁-C₄)alkoxy, cyano, (C₃-C₁₈)cycloalkyl, (C₃-C₁₈)cycloalkoxy or (C₃-C₁₈)cycloalkyl-methoxy;
R₃ is SO₂-Y, COZ, CN or (C₁-C₆)hydroxyalkyl, wherein:
Y is (C₁-C₆)alkyl, benzyl or phenyl;
Z is -NR₆R₇, (C₁-C₆)alkyl, benzyl or phenyl;
R₆ is H, (C₁-C₄)alkyl, (C₃-C₁₈)cycloalkyl, (C₂-C₅)alkanoyl, benzyl or phenyl, each of which can be optionally substituted with halo, amino or (C₁-C₄)alkyl-amino;
R₇ is H or (C₁-C₄)alkyl;
R₄ and R₅ are taken together to provide -NH-CH₂-R₈-, NH CO-R₈-, or -N=CH-R₈-, wherein:
   R₈ is CH₂, O, NH, CH=CH, CH=N, or N=CH; or
   one of R₄ and R₅ is H, and the other of R₄ and R₅ is imidazoyl, pyrrolyl, oxadiazolyl, triazolyl, or a structure of formula (A), 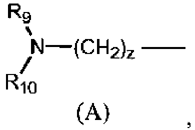 wherein:
   z is 0 or 1;
   R₉ is: H; (C₁-C₄)alkyl, (C₃-C₁₈)cycloalkyl, (C₂-C₅)alkanoyl, or (C₄-C₆)cycloalkanoyl, optionally substituted with halo, amino, (C₁-C₄)alkyl-amino, or (C₁-C₄)dialkyl-amino; phenyl; benzyl; benzyl; (C₂-C₅)alkoxycarbonyl; (C₃-C₅)alkoxyalkylcarbonyl; N-morpholinocarbonyl; carbamoyl; N-substituted carbamoyl substituted with (C₁-C₄)alkyl; or methylsulfonyl; and
   R₁₀ is H, (C₁-C₄)alkyl, methylsulfonyl, or (C₃-C₅)alkoxyalkylcarbonyl-, or
   R₉ and R₁₀ are taken together to provide -CH=CH-CH=H-, -CH=CH-N=CH-, or (C₁-C₂)alkylidene, optionally substituted with amino, (C₁-C₄)alkyl-amino, or (C₁-C₄)dialkyl-amino; or
   R₄ and R₅ are both structures of formula (A).
   In one embodiment, z is not 0 when *(i)* R³ is -SO₂-Y, -COZ, or -CN and *(ii)* one of R⁴ or R⁵ is hydrogen. In another embodiment, R⁹ and R¹⁰, taken together, is -CH=CH-CH=CH-, -CH=CH-N=CH-, or (C₁-C₂)alkylidene substituted by amino, (C₁-C₄)alkylamino, or (C₁-C₄)dialkyl-amino. In another embodiment, R₄ and R₅ arc both structures of formula (A).

In some embodiments, compounds include those of formula: 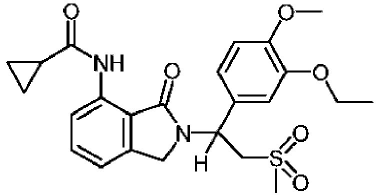 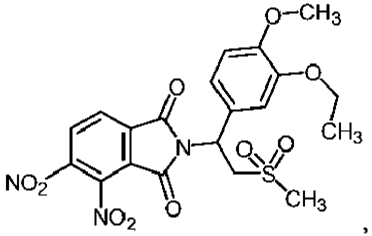 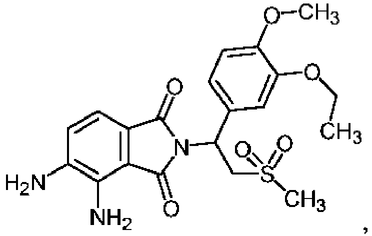 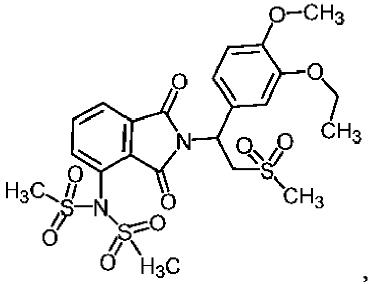 and 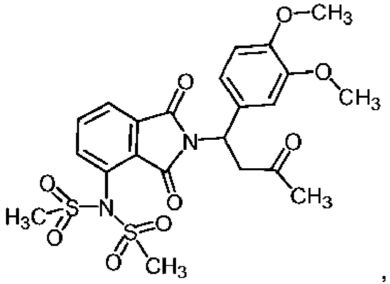 and pharmaceutically acceptable salts, solvates, hydrates, clathrates, stereoisomers, and prodrugs thereof.

Further examples include, but are not limited to: 2-[1-(3-Ethoxy-4-methoxyphenyl)-2-methylsulfonylethyl]-4,5-dinitroisoindoline-1,3-dione; 2-[1-(3-Ethoxy-4-methoxyphenyl)-2-methylsulfonylethyl]-4,5-diaminoisoindoline-1,3-dione; 7-[1-(3- Ethoxy-4-methoxyphenyl)-2-methylsulfonylethyl]-3-pyrrolino[3,4-e]benzimidazole-6,8-dione; 7-[1-(3-Ethoxy-4-methoxyphenyl)-2-methylsulfonylethyl]hydro-3-pyrrolino[3,4-c]benzimidazole-2,6,8-trione; 2-[1-(3-Ethoxy-4-methoxyphenyl)-2-methylsulfonylethyl]-3-pyrrolino[3,4-f]quinoxaline-1,3-dione; Cyclopropyl-N-{2-[1-(3-ethoxy-4-methoxyphenyl)-2-methylsulfonylethyl]-1,3-dioxoisoindolin-4-yl}carboxamide; 2-Chloro-N-{2-[1-(3-ethoxy-4-methoxyphenyl)-2-methylsulfonylethyl]-1,3-dioxoisoindolin-4-yl}acetamide; 2-Amino-N-{2-[1-(3-ethoxy-4-methoxyphenyl)-2-methylsulfonylethyl]-1,3-dioxoisoindolin-4-yl}acetamide; 2-N,N-Dimethylamino-N-{2-[-(3-ethoxy-4-methoxyphenyl)-2-methylsulfonylethyl]-1,3-dioxoisoindolin-4-yl}acetamide; N-{2-[1-(3-ethoxy-4-methoxyphenyl)-2-methylsulfonylethyl]-1,3-dioxoisoindolin-4-yl}-2,2,2-trifluoroacetamide; N-{2-[1-(3-Ethoxy-4-methoxyphenyl)-2-methylsulfonylethyl]-1,3-dioxoisoindolin-4-yl}methoxycarboxamide; 4-[1-Aza-2-(dimethylamino)vinyl]-2-[1-(3-ethoxy-4-methoxyphenyl)-2-methylsulfonylethyl]isoindoline-1,3-dione; 4-[1-Aza-2-(dimethylamino)prop-1-enyl]-2-[1-(3-ethoxy-4-methoxyphenyl)-2-methylsulfonylethyl]isoindoline-1,3-dione; 2-[1-(3-Ethoxy-4-methoxyphenyl)-2-methylsulfonylethyl]-4-(5-methyl-1,3,4-oxadiazol-2-yl)isoindoline-1,3-dione; 2-[1-(3-Ethoxy-4-methoxyphenyl)-2-methylsulfonylethyl]-4-pyrrolylisoindoline-1,3-dione; 4-(Aminomethyl)-2-[1-(3-ethoxy-4-methoxyphenyl)-2-methylsulfonylethyl]-isoindoline-1,3-dione; 2-[1-(3-Ethoxy-4-methoxyphenyl)-2-methylsulfonylethyl]-4-(pyrrolylmethyl)isoindoline-1,3-dione; N-{2-[1-(3-ethoxy-4-methoxyphenyl)-3-hydroxybutyl]-1,3-dioxoisoindolin-4-yl}acetamide; N-{2-[1-(3-Ethoxy-4-methoxyphenyl)-3-oxobutyl]-1,3-dioxoisoindolin-4-yl}acetamide; N-{2-[1R-(3-ethoxy-4-methoxyphenyl)-3-hydroxybutyl]-1,3-dioxoisoindolin-4-yl}acetamide; N-{2-[1R-(3-ethoxy-4-methoxyphenyl)-3-oxobutyl]-1,3-dioxoisoindolin-4-yl}acetamide; N-{2-[1S-(3-Ethoxy-4-methoxyphenyl)-3-hydroxybutyl]-1,3-dioxoisoindolin-4-yl}acetamide; N-{2-[1S-(3-ethoxy-4-methoxyphenyl)-3-oxobutyl]-1,3-dioxoisoindolin-4-yl}acetamide; 4-Amino-2-[1-(3-ethoxy-4-methoxyphenyl)-3-hydroxybutylisoindoline-1,3-dione; 4-Amino-2-[1-(3-ethoxy-4-methoxyphenyl)-3-oxobutyl]isoindoline-1,3-dione; 2-[1-(3-Ethoxy-4-methoxyphenyl)-3-oxobutyl]-4-pyrrolylisoindoline-1,3-dione; 2-Chloro-N-{2-[1-(3-ethoxy-4-methoxyphenyl)-3-oxobutyl]-1,3-dioxoisoindol-4-yl}acetamide; 2-(Dimethylamino)-N-{2-[1-(3-ethoxy-4-methoxyphenyl)-3-oxobutyl]-1,3-dioxoisoindolin-4-yl}acetamide; 4-Amino-2-[1R-(3-ethoxy-4-methoxyphenyl)-3-hydroxybutyl]isoindoline-1,3-dione; 4-Amino-2-[1R-(3-ethoxy-4-methoxyphenyl)-3-oxobutyl]isoindoline-1,3-dione; 2-[1R-(3-ethoxy-4-methoxyphenyl)-3-oxobutyl]-4-pyrrolylisoindoline-1,3-dione; 2-(Dimethylamino)-N-{2-[1R-(3-ethoxy-4-methoxyphenyl)-3-oxobutyl]-1,3-dioxoisoindolin-4-yl} acetami de; Cyclopentyl-N-{2-[1-(3-ethoxy-4-methoxyphenyl)-2-(methylsulfonyl)ethyl]-1,3-dioxoisoindolin-4-yl}carboxamide; 3-(Dimethylamino)-N-{2-[1-(3-ethoxy-4-methoxyphenyl)-2-(methylsulfonyl)ethyl]-1,3-dioxoisoindolin-4-yl}propanamide; 2-(Dimethylamino)-N-{2-[1-(3-ethoxy-4-methoxyphenyl)-2-(methylsulfonyl)ethyl]-1,3-dioxoisoindolin-4-yl}propanamide; N-{2-[(1R)-1-(3-ethoxy-4-methoxyphenyl)-2-(methyklsulfonyl)ethyl]-1,3-dioxoisoindolin-4-yl}-2-(dimethylamino)acetamide; N-{2-[(1S)-1-(3-ethoxy-4-methoxyphenyl)-2-(methylsulfonyl)ethyl]-1,3-dioxoisoindolin-4-yl}-2-(dimethylamino)acetamide; 4-{3-[(Dimethylamino)methyl]pyrrolyl}-2-[1-(3-ethoxy-4-methoxyphenyl)-2-(methylsulfonyl)ethyl]isoindoline-1,3-dione; Cyclopropyl-N-{2-[(1S)-1-(3-ethoxy-4-methoxyphenyl)-2-(methylsulfonyl)ethyl]-1,3-dioxoisoindolin-4-yl}carboxamide; 2-[1-(3,4-dimethoxyphenyl)-2-(methylsulfonyl)ethyl]-4-pyrrolylisoindoline-1,3-dione; N-{2-[1-(3,4-dimethoxyphenyl)-2-(methylsulfonyl)ethyl]-1,3-dioxoisoindolin-4-yl}-2-(dimethylamino)acetamide; Cyclopropyl-N-{2-[1-(3,4-dimethoxyphenyl)-2-(methylsulfonyl)ethyl]-1,3-dioxoisoindolin-4-yl}carboxamide; Cyclopropyl-N-{2-[1-(3-ethoxy-4-methoxyphenyl)-2-(methylsulfonyl)ethyl]-3-oxoisoindolin-4-yl}carboxamide; 2-(Dimethylamino)-N-{2-[1-(3-ethoxy-4-methoxyphenyl)-2-(methylsulfonyl)ethyl]-3-oxoisoindolin-4-yl}acetamide; Cyclopropyl-N-{2-[(1S)-1-(3-ethoxy-4-methoxyphenyl)-2-(methylsulfonyl)ethyl]-3-oxoisoindolin-4-yl}carboxamide; Cyclopropyl-N-{2-[(1R)-1-(3-ethoxy-4-methoxyphenyl)-2-(methylsulfonyl)ethyl]-3-oxoisoindolin-4-yl}carboxamide; (3R)-3-[7-(Acetylamino)-1-oxoisoindolin-2-yl]-3-(3-ethoxy-4-methoxyphenyl)-N,N-dimethylpropanamide; (3R)-3-[7-(Cyclopropylcarbonylamino)-1-oxoisoindolin-2-yl]-3-(3-ethoxy-4-methoxyphenyl)-N,N-dimethylpropanamide; 3-{4-[2-(Dimethylamino)acetylamino]-1,3-dioxoisoindolin-2-yl}-3-(3-ethoxy-4-methoxyphenyl)-N,N-dimethylpropanamide; (3R)-3-[7-(2-Chloroacetylamino)-1-oxoisoindolin-2-yl]-3-(3-ethoxy-4-methoxy-phenyl)-N,N-dimethylpropanamide; (3R)-3-{4-[2-(dimethylamino)acetylamino]-1,3-dioxoisoindolin-2-yl}-3-(3-ethoxy-4-methoxyphenyl)-N,N-dimethylpropanamide; 3-(1,3-Dioxo-4-pyrrolylisoindolin-2-yl)-3-(3-ethoxy-4-methoxyphenyl)-N,N-dimethylpropanamide; 2-[1-(3-Ethoxy-4-methoxyphenyl)-2-(methylsulfonyl)ethyl]-4-(imidazolyl-methyl)isoindoline-1,3-dione; N-({2-[1-(3-Ethoxy-4-methoxyphenyl)-2-(methylsulfonyl)ethyl]-1,3-dioxoisoindolin-4-yl}methyl)acetamide; 2-Chloro-N-({2-[1-(3-ethoxy-4-methoxyphenyl)-2-(methylsulfonyl)ethyl]-1,3 dioxoisoindolin-4-yl}methyl)acetamide; 2-(Dimethylamino)-N-({2-[1-(3-ethoxy-4-methoxyphenyl)-2-(methylsulfonyl)ethyl]-1,3-dioxoisoindolin-4-yl}methyl)acetamide; 4-[Bis(methylsulfonyl)amino]-2-[1-(3-ethoxy-4-methoxyphenyl)-2-(methylsulfonyl)ethyl]isoindoline-1,3-dione; 2-[1-(3-Ethoxy-4-methoxyphenyl)-2-(methylsulfonyl)ethyl]-4-[(methylsulfonyl)amino]isoindoline-1,3-dione; N-{2-[1-(3-Ethoxy-4-methoxyphenyl)-3-hydroxypentyl]-1,3-dioxoisoindolin-4-yl}acetamide; N-{2-[1-(3-Ethoxy-4-methoxyphenyl)-3-oxopentyl]1,3-dioxoisoindolin-4-yl}acetamide; 2-[(1R)-1-(3-Ethoxy-4-methoxyphenyl)-3-hydroxybutyl]-4-(pyrrolylmethyl)isoindoline-1,3-dione; 2-[(1R)-1-(3-Ethoxy-4-methoxyphenyl)-3-oxobutyl]-4-(pyrrolylmethyl)isoindoline-1,3-dione; N-{2-[1-(3-Cyclopentyloxy-4-methoxyphenyl)-3-hydroxybutyl]-1,3-dioxoisoindolin-4-yl}acetamide; N-{2-[1-(3-Cyclopentyloxy-4-methoxyphenyl)-3-oxobutyl]-1,3-dioxoisoindolin-4-yl}acetamide; 2-[1-(3-Cyclopentyloxy-4-methoxyphenyl)-3-oxobutyl]-4-pyrrolylisoindoline-1,3-dione; 2-[1-(3,4-Dimethoxyphenyl)-3-oxobutyl]-4-[bis(methylsulfonyl)amino]isoindoline-1,3-dione; and pharmaceutically acceptable salts, solvates, hydrates, clathrate, stereoisomers, and prodrugs thereof.

Still other PDE4 modulators include, but are not limited to, imido and amido substituted acylhydroxamic acids (for example, (3-(1,3-dioxoisoindoline-2-yl)-3-(3-ethoxy-4-methoxyphcnyl) propanoylamino) propanoate disclosed in WO 01/45702 and U.S. patent no. 6,699,899, which are incorporated herein by reference. Examples include compounds of formula: 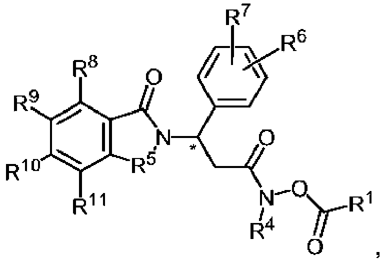 and pharmaceutically acceptable salts, solvates, hydrates, clathrates, stereoisomers, and prodrugs thereof, wherein:
the carbon atom designated * constitutes a center of chirality,
R⁴ is hydrogen or -(C=O)-R¹²,
each of R¹ and R¹², independently of each other, is alkyl of 1 to 6 carbon atoms, phenyl, benzyl, pyridyl methyl, pyridyl, imidazoyl, imidazolyl methyl, or
CHR*(CH₂)ₙNR*R⁰,
wherein R* and R⁰, independently of the other, are hydrogen, alkyl of 1 to 6 carbon atoms, phenyl, benzyl, pyridyl methyl, pyridyl, imidazoyl or imidazolylmethyl, and n = 0, 1, or 2;
R⁵ is C=O, CH₂, CH₂-CO-, or SO₂;
each of R⁶ and R⁷, independently of the other, is nitro, cyano, trifluoromethyl, carbethoxy, carbomethoxy, carbopropoxy, acetyl, carbamoyl, acetoxy, carboxy, hydroxy, amino, alkyl of 1 to 6 carbon atoms, alkoxy of 1 to 6 carbon atoms, cycloalkoxy of 3 to 8 carbon atoms, halo, bicycloalkyl of up to 18 carbon atoms, tricycloalkoxy of up to 18 carbon atoms, 1-indanyloxy, 2-indanyloxy, C₄-C₈-cycloalkylidenemethyl, or C₃-C₁₀-alkylidenemethyl;
each of R⁸, R⁹, R¹⁰, and R¹¹, independently of the others, is
   (i) hydrogen, nitro, cyano, trifluoromethyl, carbethoxy, carbomcthoxy, carbopropoxy, acetyl, carbamoyl, acetoxy, carboxy, hydroxy, amino, alkylamino, dialkylamino, acylamino, alkyl of 1 to 10 carbon atoms, alkoxy of 1 to 10 carbon atoms, halo, or
   (ii) one of R⁸, R⁹, R¹⁰, and R¹¹ is acylamino comprising a lower alkyl, and the remaining of R⁸, R⁹, R¹⁰, and R¹¹ are hydrogen, or
   (iii) hydrogen if R⁸ and R⁹ taken together are benzo, quinoline, quinoxaline, benzimidazole, benzodioxole, 2-hydroxybenzimidazole, methylenedioxy, dialkoxy, or dialkyl, or
   (iv) hydrogen if R¹⁰ and R¹¹, taken together are benzo, quinoline, quinoxaline, benzimidazole, benzodioxole, 2-hydroxybenzimidazole, methylenedioxy, dialkoxy, or dialkyl, or
   (v) hydrogen if R⁹ and R¹⁰ taken together arc benzo.

Still other PDE4 modulators include, but are not limited to, 7-amido-isoindolyl compounds disclosed in U.S. patent no. 7,034,052, which is incorporated herein by reference. Examples include compounds of formula: 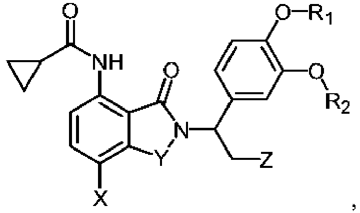 and pharmaceutically acceptable salts, solvates, hydrates, clathrates, stereoisomers, and prodrugs thereof, wherein:
Y is -C(O)-, -CH₂, -CH₂C(O)-or SO₂;
X is H;
Z is (C₀₋₄-alkyl)-C(O)R³, C₁₋₄-alkyl, (C₀₋₄₋alkyl)-OH, (C₁₋₄-alkyl)-O(C₁₋₄-alkyl), (C₁₋₄-alkyl)-SO₂(C₁₋₄-alkyl), (C₀₋₄-alkyl)-SO(C₁₋₄-alkyl), (C₀₋₄-alkyl)-NH₂, (C₀₋₄-alkyl)-N(C₁₋₈akyl)₂, (C₀₋₄-alkyl)-N(H)(OH), or CH₂NSO₂(C₁₋₄-alkyl);
R₁ and R₂ are independently C₁₋₈-alkyl, cycloalkyl, or (C₁₋₄-alkyl)cycloalkyl;
R³ is, NR⁴ R⁵, OH, or O-(C₁₋₈-alkyl);
R⁴ is H;
R⁵ is -OH, or -OC(O)R⁶; and
R⁶ is C₁₋₈-alkyl, amino-(C₁₋₈-alkyl), (C₁₋₈-alkyl)-(C₃₋₆-cycloalkyl), C₃₋₆-cycloalkyl, phenyl, benzyl, or aryl.

In other embodiments, provided herein is a compound of the following formula, or a pharmaceutically acceptable salt, solvate, hydrate, stereoisomer, clathrate, or prodrug thereof: 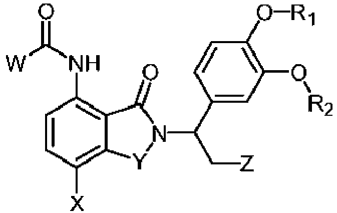 wherein:
Y is -C(O)-, -CH₂, -CH₂C(O)-, or SO₂;
X is halogen, -CN, -NR₇R₈, -NO₂, or -CF₃;
Z is (C₀₋₄alkyl)-SO₂(C₁₋₄-alkyl), -(C₀₋₄-alkyl)-CN, -(C₀₋₄-alkyl)-C(O)R³, C₁₋₄-alkyl, (C₀₋₄-alkyl)OH, (C₀₋₄-alkyl)O(C₁₋₄-alkyl), (C₀₋₄-alkyl)SO(C₁₋₄-alkyl), (C₀₋₄-alkyl)NH₂, (C₀₋₄-alkyl)N(C₁₋₈-alkyl)₂, (C₀₋₄-alkyl) N(H)(OH), (C₀₋₄-alkyl)-dichloropyridine or (C₀₋₄-alkyl)NSO₂(C₁₋₄-alkyl);
W is -C₃₋₆-cycloalkyl, -(C₁₋₈-alkyl)-(C₃₋₆-cycloalkyl), -(C₀₋₈-alkyl)-(C₃₋₆-cycloalkyl)-NR₇R₈, [C₀₋₈-alkyl)-NR₇R₈, (C₀₋₄alkyl)-CHR₉-(C₀₋₄alkyl)-NR₇R₈;
R₁ and R₂ are independently C₁₋₈-alkyl, cycloalkyl, or (C₁₋₄-alkyl)cycloalkyl;
R³ is C₁₋₈-alkyl, NR⁴R⁵, OH, or O-(C₁₋₈-alkyl);
R⁴ and R⁵ arc independently H, C₁₋₈-alkyl, (C₀₋₈-alkyl)-(C₃₋₆-cycloalkyl), OH, or-OC(O)R⁶;
R⁶ is C₁₋₈-alkyl, (C₀₋₈-alkyl)-(C₃₋₆-cycloalkyl), amino-(C₁₋₈-alkyl), phenyl, benzyl, or aryl;
R₇ and R₈ are each independently H, C₁₋₈-alkyl, (C₀₋₈-alkyl)-(C₃₋₆-cycloalkyl), phenyl, benzyl, aryl, or can be taken together with the atom connecting them to form a 3 to 7 membered heterocycloalkyl or heteroaryl ring;
is C₁₋₄ alkyl, (C₀₋₄alkyl)aryl, (C₀₋₄alkyl)-(C₃₋₆-cycloalkyl), (C₀₋₄alkyl)-heterocylcle.

In one embodiment, W is

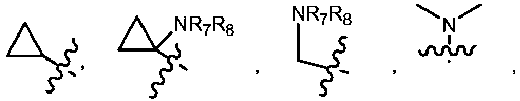

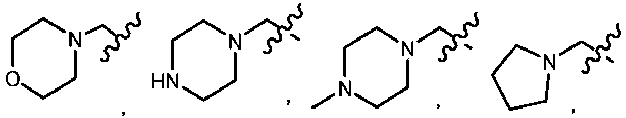

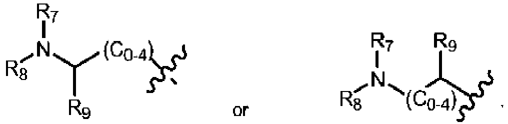

In another embodiment, representative compounds include those of formula: 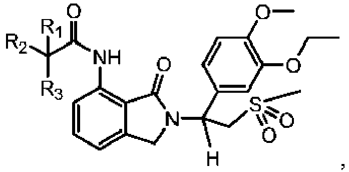 and pharmaceutically acceptable salts, solvates, hydrates, clathrates, stereoisomers, and prodrugs thereof, wherein:
R₁, R₂ and R₃ are independently H or C₁₋₈-alkyl, with the proviso that at least one of R₁, R₂ and R₃ is not H.

Still other PDE4 modulators include, but are not limited to, isoindoline compounds disclosed in U.S. publication no. 2006/0025457A1, published February 2, 2006, which is incorporated herein by reference. Representative compounds include those listed in Table 1 below, and pharmaceutically acceptable prodrugs, salts, solvates, hydrates, clathrates, and stereoisomers thereof:

**Table 1.**

| **No.** | **Structure** | **No.** | **Structure** |
|---|---|---|---|
| 1 | 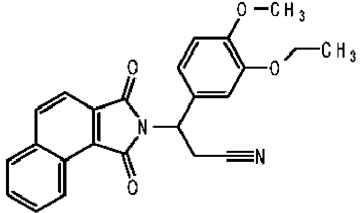 | 2 | 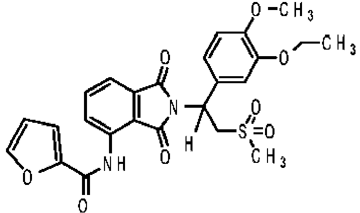 |
| 3 | 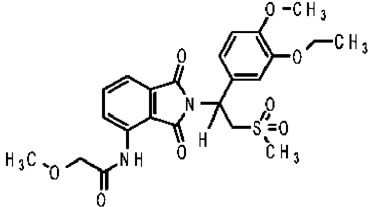 | 4 | 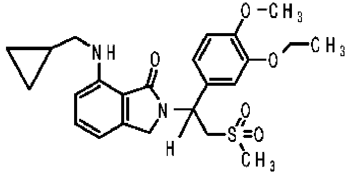 |
| 5 | 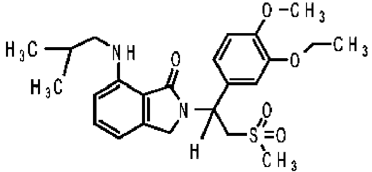 | 6 | 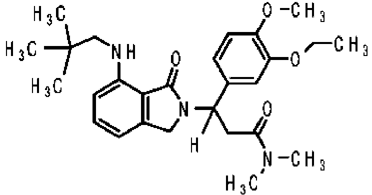 |
| 7 | 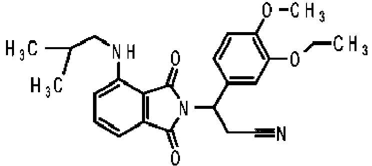 | 8 | 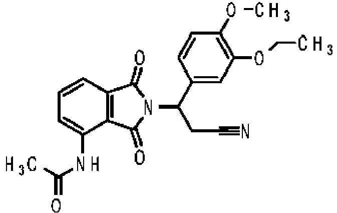 |
| 9 | 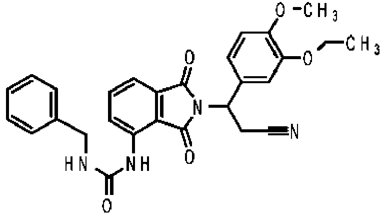 | 10 | 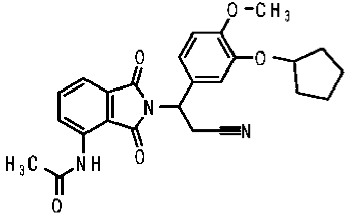 |
| 11 | 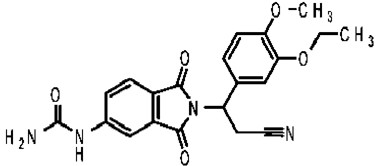 | 12 | 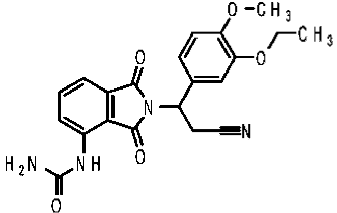 |
| 13 | 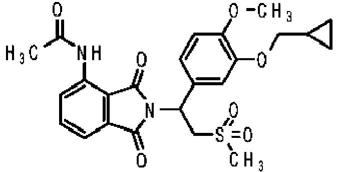 | 14 | 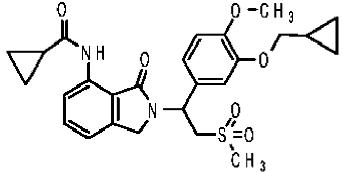 |
| 15 | 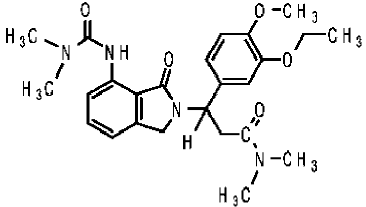 | 16 | 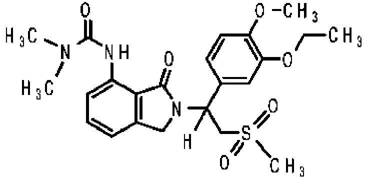 |

In another embodiment, also provided herein are 2-[1-(3-ethoxy-4-methoxyphenyl)-2-methylsulfonylethyl]-4,5-dinitroisoindoline-1,3-dione, or a pharmaceutically acceptable salt, solvate, hydrate, clathrate, stereoisomer, or prodrug thereof. In one embodiment, provided herein is a hydrochloride salt of 2-[1-(3-ethoxy-4-methoxyphenyl)-2-methylsulfonylethyl]-4,5-dinitroisoindoline-1,3-dione.

Still other PDE4 modulators include, but are not limited to, isoindoline compounds disclosed in U.S. patent no. 7,244,259, which is incorporated herein by reference. Representative compounds include cyclopropanecarboxylic acid {2-[1-(3-ethoxy-4-methoxy-phenyl)-2-[1,3,4]oxadiazol-2-yl-ethyl]-3-oxo-2,3-dihydro-1H-isoindol-4-yl}-amide, which has the following chemical structure, and pharmaceutically acceptable salts, solvates, hydrates, clathrates, prodrugs, and stereoisomers thereof:

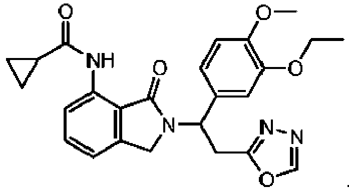

Still other PDE4 modulators include, but are not limited to, N-alkyl-hydroxamic acid-isoindolyl compounds disclosed in U.S. patent no. 6,911,464, which is incorporated herein by reference. Representative compounds include those of formula: 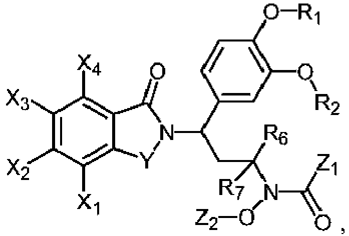 and pharmaceutically acceptable salts, solvates, hydrates, clathrates, stereoisomers, and prodrugs thereof, wherein:
Y is -C(O)-, -CH₂, -CH₂C(O)- or SO₂;
R₁ and R₂ are independently C₁₋₈-alkyl, CF₂H, CF₃, CH₂CHF₂, cycloalkyl, or (C₁₋₈-alkyl)cycloalkyl;
Z₁ is H, C₁₋₆-alkyl, -NH₂ -NR₃R₄ or OR₅;
Z₂ is H or C(O)R₅;
X₁, X₂, X₃ and X₄ arc each independent H, halogen, NO₂, OR₃, CF₃, C₁₋₆-alkyl, (C₀₋₄ alkyl)-(C₃₋₆-cycloalkyl), (C₀₋₄-alkyl)-N-(R₈R₉), (C₀₋₄-alkyl)-NHC(O)-(R₈), (C₀₋₄-alkyl)-NHC(O)CH(R₈)(R₉), (C₀₋₄-alkyl)-NHC(O)N(R₈R₉), (C₀₋₄-alkyl)-NHC(O)O(R₈), (C₀₋₄-alkyl)-O-R₈, (C₀₋₄-alkyl)-imidazolyl, (C₀₋₄-alkyl)-pyrrolyl, (C₀₋₄-alkyl) oxadiazolyl, (C₀₋₄-alkyl)-triazolyl or (C₀₋₄-alkyl)-heterocycle;
R₃, R₄, and R₅ are each independently H, C₁₋₆-alkyl, O-C₁₋₆-alkyl, phenyl, benzyl, or aryl;
R₆ and R₇ are independently H or C₁₋₆-alkyl; and
R₈ and are each independently H, C₁₋₉-alkyl, C₃₋₆-cycloalkyl, (C₁₋₆-alkyl)-(C₃₋₆-cycloalkyl), (C₀₋₆-alkyl)-N(R₄R₅), (C₁₋₆-alkyl)-OR₅, phenyl, benzyl, aryl, piperidinyl, piperizinyl, pyrolidinyl, morpholino, or C₃₋₇-heterocycloalkyl.

Still other PDE4 modulators include, but are not limited to, diphenylethylene compounds disclosed in U.S. patent no. 7,312,241, which is incorporated herein by reference. Representative compounds include those of formula: 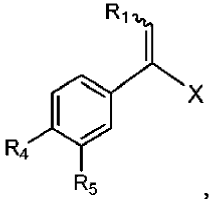 and pharmaceutically acceptable salts, solvates, hydrates, clathrates, stereoisomers, and prodrugs thereof, wherein:
R₁ is -CN, lower alkyl, -COOH, -C(O)-N(R₉)₂, -C(O)-lower alkyl, -C(O)-benzyl, - C(O)O-lower alkyl, -C(O)O-benzyl;
R⁴ is -H, -NO₂, cyano, substituted or unsubstituted lower alkyl, substituted or unsubstituted alkoxy, halogen, -OH, -C(O)(R₁₀)₂, -COOH, NH₂, -OC(O)-N(R₁₀)₂;
R⁵ is substituted or unsubstituted lower alkyl, substituted or unsubstituted alkoxy, or substituted or unsubstituted alkenyl;
X is substituted or unsubstituted phenyl, substituted or unsubstituted pyridine, substituted or unsubstituted pyrrolidine, substituted or unsubstituted imidizole, substituted or unsubstituted naphthalene, substituted or unsubstituted thiophene, or substituted or unsubstituted cycloalkyl;
each occurrence of R₉ is independently -H or substituted or unsubstituted lower alkyl; and
each occurrence of R₁₀ is independently -H or substituted or unsubstituted lower alkyl.

In another embodiment, representative compounds include those of formula: 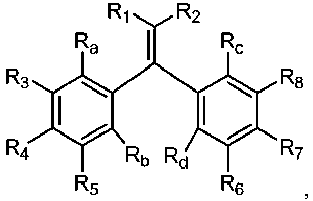 and pharmaceutically acceptable salts, solvates, hydrates, clathrates, stereoisomers, and prodrugs thereof, wherein:
R₁ and R₂ are independently -H, -CN, substituted or unsubstituted lower alkyl, substituted or unsubstituted alkenyl, substituted or unsubstituted alkynyl, -COOH, -C(O)-lower alkyl, -C(O)O-lower alkyl, -C(O)-N(R₉)₂, substituted or unsubstituted aryl, or substituted or unsubstituted heterocycle;
each occurrence of Rₐ, R_{b}, R_{c} and R_{d} is independently -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R₃ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂, or R₃ with either Rₐ or with R₄, together form -O-C(R₁₆R₁₇)-O- or -O-(C(R₁₆R₁₇))₂-O-;
R⁴ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R⁵ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R⁶ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂;
R₇ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₇, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₃-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂-;
R₈ is -H, substituted or unsubstituted lower alkyl, substituted or unsubstituted aryl, substituted or unsubstituted heterocycle, substituted or unsubstituted cycloalkyl, substituted or unsubstituted alkoxy, halogen, cyano, -NO₂, -OH, -OPO(OH)₂, -N(R₉)₂, -OC(O)-R₁₀, -OC(O)-R₁₀-N(R₁₀)₂, -C(O)N(R₁₀)₂, -NHC(O)-R₁₀, -NHS(O)₂-R₁₀, -S(O)₂-R₁₀, -NHC(O)NH-R₁₀, -NHC(O)N(R₁₀)₂, -NHC(O)NHSO₂-R₁₀, -NHC(O)-R₁₀-N(R₁₀)₂, -NHC(O)CH(R₁₀)(N(R₉)₂) or -NHC(O)-R₁₀-NH₂, or R₈ with either R_{c} or with R₇, together form -O-C(R₁₆R₁₇)-O- or -O-(C(R₁₆R₁₇))₂-O-;
each occurrence of R₉ is independently -H, substituted or unsubstituted lower alkyl, or substituted or unsubstituted cycloalkyl;
each occurrence of R₁₀ is independently substituted or unsubstituted lower alkyl, substituted or unsubstituted cycloalkyl, substituted or unsubstituted aryl, substituted or unsubstituted lower hydroxyalkyl, or R₁₀ and a nitrogen to which it is attached form a substituted or unsubstituted heterocycle, or R₁₀ is -H where appropriate; and
each occurrence of R₁₆ and R₁₇ is independently -H or halogen.

In another embodiment, provided herein is 3-(3,4-dimethoxyphenyl)-3-(1-oxo-1,3-dihydroisoindol-2-yl)propionic acid methyl ester: 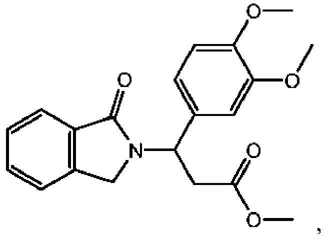 or a pharmaceutically acceptable salt, solvate, stereoisomer, or prodrug thereof.

In one embodiment, provided herein arc 2-[1-(3-ethoxy-4-methoxyphenyl)-2-methylsulfonylethyl]-4-acetylaminoisoindoline-1,3-dione and cyclopropyl-N-{2-[1-(3-ethoxy-4-methoxyphenyl)-2-(methylsulfonyl)ethyl]-3-oxoisoindolin-4-yl}carboxamide, which respectively have the following structures: 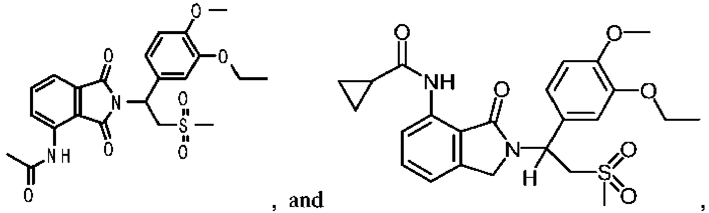 or a pharmaceutically acceptable salt, solvate or prodrug thereof. In another embodiment, stereoisomers of these compounds arc also encompassed.

Compounds provided herein can either be commercially purchased or prepared according to the methods described in the patents or patent publications disclosed herein. Further, optically pure compositions can be asymmetrically synthesized or resolved using known resolving agents or chiral columns as well as other standard synthetic organic chemistry techniques.

Various PDE4 modulators contain one or more chiral centers, and can exist as racemic mixtures of enantiomers or mixtures of diastereomers. In one embodiment, provided herein is the use of stereomerically pure forms of such compounds, as well as the use of mixtures of those forms. For example, mixtures comprising equal or unequal amounts of the enantiomers of PDE4 modulators may be used in methods and compositions provided herein. The purified (R) or (S) enantiomers of the specific compounds disclosed herein may be used substantially free of its other enantiomer.

It should be noted that if there is a discrepancy between a depicted structure and a name given that structure, the depicted structure is to be accorded more weight. In addition, if the stereochemistry of a structure or a portion of a structure is not indicated with, for example, bold or dashed lines, the structure or portion of the structure is to be interpreted as encompassing all stereoisomers of it.

### 4.3 METHODS OF TREATMENT, PREVENTION AND MANAGEMENT

Provided herein are methods of treating, preventing, and/or managing TB and other related disorders. As used herein, the treatment, prevention, and/or management of "TB" encompasses the treatment, prevention, and/or management of all forms of TB and symptoms associated therewith. Examples of TB include, but are not limited to, pulmonary TB and extrapulmonary TB (remote TB lesions) such as, but not limited to, genitourinary TB (e.g., kidney TB), tubcculous meningitis, military TB, tuberculous peritonitis, tuberculous pericarditis, tuberculous lymphadentitis, TB of bones and joints, gastrointestinal TB, and TB of the liver.

In one embodiment, provided herein are methods of treating, preventing, and/or managing the symptoms associated with TB. Examples include, but are not limited to, cough, dyspnea, hilar lymphadenopathy, segmental atelectasis, swelling of the nodes, lobar atelectasis, pulmonary caviation, fever, unremitting headache, nausea, drowsiness, stupor, coma, stiff neck, weakness, and malaise.

Also provided herein are methods of treating, preventing, and/or managing other disorders related to TB. Such disorders often include other mycobacterial infections, symptom of which resemble those of TB. Examples include, but arc not limited to, disorders caused by *M. avium* complex (MAC; *M. avium* and *M. intracellulare*), *M. kansasii, M. xenopy, M. marinum, M. ulcerans, M. leprae,* and *M fortuitum* complex (*M*. *fortuitum* and *M. chelonei*). Examples of disorders caused by these mycobacteria include, but arc not limited to, pulmonary diseases, lymphadenitis, cutaneous diseases, wounds, and foreign body infections.

In some embodiments, the treatment, prevention and/or management of other granulomatous diseases are also encompassed herein. Examples of such diseases include, but are not limited to: infectious agents caused diseases such as histoplasmosis, cryptococcus, schitosomiasis, and leishmaniasis; allergic reactions caused diseases such as berylliosis; non-infectious agents caused diseases such as aspiration pneumonia and foreign body reaction; genetically caused diseases such as chronic granulomatous disease; and diseases of unknown causes such as sarcoidosis, Crohn's disease and cat-scratch fever.

In some embodiments, methods provided herein comprise administering one or more PDE4 modulators, or a pharmaceutically acceptable salt, solvate, stereoisomer, or prodrug thereof to a subject (*e.g.,* a human) suffering, or likely to suffer, from TB or other related disorders. In certain embodiments, the PDE4 modulators are administered in combination with conventional chemotherapeutic agents currently known for the treatment, prevention, and/or management of TB and other disorders. Examples of such agents include, but arc not limited to, isoniazid, rifampin, pyrazinamide, streptomycin, ethambutol, capreomycin, ethionamide, cycloserine, levafloxacin, ciprofloxacin, amikacin, moxifloxicin, p-aminosalicylic acid, kanamycin, viomycin, enciomycin, protionamide, rifabutin, clarithromycin, linezolid, thioacetazone, arginine, vitamin B, and corticosteroids.

In other embodiments, the PDE4 modulators are administered in combination with conventional therapy currently known for the treatment, prevention, and/or management of TB and other disorders. Examples of such therapy include, but are not limited to, surgical resection.

In one embodiment, the PDE4 modulator used in connection with methods provided herein is {2-[1-(3-ethoxy-4-methoxyphenyl)-2-methylsulfonylethyl]-4-acetylaminoisoindoline-1,3-dione}, cyclopropyl-N-{2-[1-(3-ethoxy-4-methoxyphenyl)-2-(methylsulfonyl)ethyl]-3oxoisoindoline-4-yl}carboxamide, or a pharmaceutically acceptable salt, solvate, or stereoisomer thereof. In one embodiment, the PDE4 modulator is (+)-{2-[1-(3-ethoxy-4-methoxyphenyl)-2-methylsulfonylethyl]-4-acetylaminoisoindoline-1,3-dione}, or a pharmaceutically acceptable salt or solvate thereof. In another embodiment, the PDE4 modulator is cyclopropyl-N- {2-[(1S)-1-(3-ethoxy-4-methoxyphenyl)-2-(methylsulfonyl)ethyl]-3-oxoisoindoline-4-yl}carboxamide, or a pharmaceutically acceptable salt or solvate thereof.

In one embodiment, a PDE4 modulator is administered orally and in single or divided daily doses in an amount of from about 1 mg to about 10,000 mg. In certain embodiments, the daily dose is administered twice daily in equally divided doses. In one embodiment, a daily dose range should be from about 1 mg to about 5,000 mg per day, from about 10 mg to about 2,500 mg per day, from about 100 mg to about 800 mg per day, from about 100 mg to about 1,200 mg per day, or from about 25 mg to about 2,500 mg per day. In managing the patient, the therapy should be initiated at a lower dose, perhaps about 1 mg to about 2,500 mg, and increased if necessary up to about 200 mg to about 5,000 mg per day as either a single dose or divided doses, depending on the patient's global response.

In one embodiment, 3-(3,4-dimethoxy-phenyl)-3-(1-oxo-1,3-dihydro-isoindol-2-yl)-propionamidc can be administered in an amount of about 400, 800, 1,200, 2,500, 5,000 or 10,000 mg a day as two divided doses. In one embodiment, 3-(3,4-dimethoxy-phenyl)-3-(1-oxo-1,3-dihydro-isoindol-2-yl)-propionamide is administered in an amount of from about 400 to about 1,200 mg/d daily, every other day, or in other syncopated regimen.

In another embodiment, 2-[1-(3-ethoxy-4-methoxyphenyl)-2-methylsulfonylethyl]-4-acetylaminoisoindoline-1,3-dione, or a stereoisomer thereof, is administered in an amount of about 1, 10, 100, 200, 400, 800, 1,200, 2,500, 5,000, or 10,000 mg a day as a single or two divided doses.

In another embodiment, cyclopropyl-N-{2-[1-(3-ethoxy-4-methoxyphenyl)-2-methylsulfonyl)ethyl]-3-oxoisoindolin-4-yl}carboxamide, or a stereoisomer thereof, is administered in an amount of about 1, 10, 100, 200, 400, 800, 1,200, 2,500, 5,000, or 10,000 mg a day as a single or two divided doses.

In one embodiment, a PDE4 modulator is administered by inhalation, and in single or divided daily doses, in an amount of from about 1 mg to about 10,000 mg. In one embodiment, the daily dose is administered twice daily in equally divided doses. In certain embodiments, a daily dose range is from about 1 mg to about 5,000 mg per day, from about 10 mg to about 2,500 mg per day, from about 100 mg to about 800 mg per day, from about 100 mg to about 1,200 mg per day, or from about 25 mg to about 2,500 mg per day. In managing the patient, the therapy may be initiated at a lower dose, perhaps about 1 mg to about 2,500 mg, and increased if necessary up to about 200 mg to about 5,000 mg per day as either a single dose or divided doses, depending on the patient's global response.

In one embodiment, 3-(3,4-dimethoxy-phenyl)-3-(1-oxo-1,3-dihydro-isoindol-2-yl)-propionamide can be administered in an amount of about 400, 800, 1,200, 2,500, 5,000 or 10,000 mg a day as two divided doses. In another embodiment, 3-(3,4-dimethoxy-phenyl)-3-(1-oxo-1,3-dihydro-isoindol-2-yl)-propionamide is administered in an amount of from about 400 to about 1,200 mg/d daily, every other day, or in other syncopated regimen.

In another embodiment, 2-[1-(3-ethoxy-4-methoxyphenyl)-2-methylsulfonylethyl]-4-acetylaminoisoindoline-1,3-dione, or a stereoisomer thereof, is administered in an amount of about 1, 10, 100, 200, 400, 800, 1,200, 2,500, 5,000, or 10,000 mg a day as a single or two divided doses.

In another embodiment, cyclopropyl-N-{2-[1-(3-ethoxy-4-methoxyphenyl)-2-methylsulfonyl)ethyl]-3-oxoisoindolin-4-yl}carboxamide, or a stereoisomer thereof, is administered in an amount of about 1, 10, 100, 200, 400, 800, 1,200, 2,500, 5,000, or 10,000 mg a day as a single or two divided doses.

### 4.3.1 Combination Therapy

In certain embodiments, provided herein arc methods which comprise administering a PDE4 modulator, or a pharmaceutically acceptable salt, solvate, hydrate, clathrate, stereoisomer, or prodrug thereof, in combination with one or more other therapeutic agents. Examples of PDE4 modulators are disclosed herein (*see*, *e.g.,* section 4.2); and examples of second active agents are also disclosed herein (*see*, *e.g.,* section 4.3).

Administration of the PDE4 modulators and the second active agents to a patient can occur simultaneously or sequentially by the same or different routes of administration. The suitability of a particular route of administration employed for a particular active agent will depend on the active agent itself (*e.g*., whether it can be administered orally without decomposing prior to entering the blood stream) and the disease being treated. In one embodiment, the route of administration for PDE4 modulators is oral. In another embodiment, the route of administration for PDE4 modulators is nasal. Routes of administration for the second active agents or ingredients provided herein arc known to those of ordinary skill in the art. *See, e.g.,* Physicians' Desk Reference, 594-597 (57th ed., 2003).

In one embodiment, the second active agent is administered orally, intravenously, intramuscularly, subcutaneously, mucosally, or transdermally and once or twice daily in an amount of from about 1 to about 3,500 mg, from about 5 to about 2,500 mg, from about 10 to about 500 mg, or from about 25 to about 250 mg.

The specific amount of the second active agent will depend on the specific agent used, the type of disease or disorder being treated or managed, the severity and stage of the disease or disorder, and the amount(s) of PDE4 modulators and any optional additional active agents concurrently administered to the patient. Conventional amounts of the second active agents can be a starting point. *See, e.g.,* Physicians' Desk Reference, (57th cd., 2003).

In one embodiment, a PDE4 modulator and a second active agent are administered to a patient (*e.g.,* a mammal such as a human), in a sequence and within a time interval such that the PDE4 modulator can act together with the other agent to provide an increased benefit than if they were administered otherwise. For example, the second active agent can be administered at the same time or sequentially in any order at different points in time; however, if not administered at the same time, they should be administered sufficiently close in time so as to provide the desired therapeutic or prophylactic effect. In one embodiment, the PDE4 modulator and the second active agent exert their effect at times which overlap. Each second active agent can be administered separately, in any appropriate form and by any suitable route. In other embodiments, the PDE4 modulator is administered before, concurrently with, or after the administration of the second active agent.

In various embodiments, the PDE4 modulator and the second active agent are administered less than about 1 hour apart, at about 1 hour apart, at about 1 hour to about 2 hours apart, at about 2 hours to about 3 hours apart, at about 3 hours to about 4 hours apart, at about 4 hours to about 5 hours apart, at about 5 hours to about 6 hours apart, at about 6 hours to about 7 hours apart, at about 7 hours to about 8 hours apart, at about 8 hours to about 9 hours apart, at about 9 hours to about 10 hours apart, at about 10 hours to about 11 hours apart, at about 11 hours to about 12 hours apart, no more than 24 hours apart or no more than 48 hours apart. In other embodiments, the PDE4 modulator and the second active agent arc administered simultaneously using the same or different routes.

In other embodiments, the PDE4 modulator and the second active agent are administered at about 2 to 4 days apart, at about 4 to 6 days apart, at about 1 week part, at about 1 to 2 weeks apart, or more than 2 weeks apart.

In certain embodiments, the PDE4 modulator and the second active agent are cyclically administered to a patient. Cycling therapy involves the administration of a first agent for a period of time, followed by the administration of a second agent and/or third agent for a period of time and repeating this sequential administration. Cycling therapy can reduce the development of resistance to one or more of the therapies, avoid or reduce the side effects of one of the therapies, and/or improve the efficacy of the treatment.

In certain embodiments, the PDE4 modulator and optionally the second active agent are administered in a cycle of less than about 3 weeks, about once every two weeks, about once every 10 days or about once every week. One cycle can comprise the administration of a PDE4 modulator and optionally the second active agent by infusion over about 90 minutes every cycle, about 1 hour every cycle, about 45 minutes every cycle. Each cycle can comprise at least 1 week of rest, at least 2 weeks of rest, at least 3 weeks of rest. The number of cycles administered is from about 1 to about 12 cycles, more typically from about 2 to about 10 cycles, and more typically from about 2 to about 8 cycles.

In yet other embodiments, the PDE4 modulator is administered in metronomic dosing regimens, either by continuous infusion or frequent administration without extended rest periods. Such metronomic administration can involve dosing at constant intervals without rest periods. Typically the PDE4 modulators, are used at lower doses. Such dosing regimens encompass the chronic daily administration of relatively low doses for extended periods of time. In preferred embodiments, the use of lower doses can minimize toxic side effects and eliminate rest periods. In certain embodiments, the PDE4 modulator is delivered by chronic low-dose or continuous infusion ranging from about 24 hours to about 2 days, to about 1 week, to about 2 weeks, to about 3 weeks to about 1 month to about 2 months, to about 3 months, to about 4 months, to about 5 months, to about 6 months. The scheduling of such dose regimens can be optimized by the skilled artisan.

In other embodiments, courses of treatment are administered concurrently to a patient, *i.e.,* individual doses of the second active agent are administered separately yet within a time interval such that the PDE4 modulator can work together with the second active agent. For example, one component can be administered once per week in combination with the other components that can be administered once every two weeks or once every three weeks. In other words, the dosing regimens are carried out concurrently even if the therapeutics are not administered simultaneously or during the same day.

The second active agent can act additively or, more preferably, synergistically with the PDE4 modulator. In one embodiment, a PDE4 modulator is administered concurrently with one or more second active agents in the same pharmaceutical composition. In another embodiment, a PDE4 modulator is administered concurrently with one or more second active agents in separate pharmaceutical compositions. In still another embodiment, a PDE4 modulator is administered prior to or subsequent to administration of a second active agent. Also provided herein is the administration of a PDE4 modulator and a second active agent by the same or different routes of administration, *e.g*., oral and parenteral. In certain embodiments, when a PDE4 modulator is administered concurrently with a second active agent that potentially produces adverse side effects including, but not limited to, toxicity and development of resistance, the second active agent can advantageously be administered at a dose that falls below the threshold that the adverse side effect is elicited.

### 4.3.2 Use With Other Management Conventional Techniques

Also provided herein arc methods of treating, preventing, and/or managing TB and other related disorders, which comprises administering a PDE4 modulator, or a pharmaceutically acceptable salt, solvate, hydrate, clathrate, stereoisomer, or prodrug thereof, in conjunction with (e.g. before, during, or after) other conventional therapies for TB and other disorders. Examples of other conventional therapies include, but are not limited to, surgical resection.

The combined use of the PDE4 modulators and other conventional therapies may provide a unique treatment regimen that is unexpectedly effective in certain patients. Without being limited by a particular theory, it is believed that PDE4 modulators may provide additive or synergistic effects when given concurrently with other conventional therapies.

### 4.4 PHARMACEUTICAL COMPOSITIONS AND SINGLE UNIT DOSAGE FORMS

Pharmaceutical compositions can be used in the preparation of individual, single unit dosage forms. Pharmaceutical compositions and dosage forms provided herein comprise a PDE4 modulator, or a pharmaceutically acceptable salt, solvate, hydrate, clathrate, stereoisomer, or prodrug thereof. Pharmaceutical compositions and dosage forms provided herein can further comprise one or more excipients.

Pharmaceutical compositions and dosage forms provided herein can also comprise one or more additional active ingredients. Consequently, pharmaceutical compositions and dosage forms provided herein comprise the compounds provided herein (*e.g.,* a PDE4 modulator, or a pharmaceutically acceptable salt, solvate, hydrate, clathrate, stereoisomer, or prodrug thereof, and a second active agent). Examples of additional therapeutic agents are disclosed herein (*see*, *e.g.,* section 4.3).

Single unit dosage forms provided herein are suitable for oral, mucosal (*e.g.,* nasal, sublingual, vaginal, buccal, or rectal), or parenteral (*e.g.,* subcutaneous, intravenous, bolus injection, intramuscular, or intraarterial), transdermal or transcutaneous administration to a patient. Examples of dosage forms include, but are not limited to: tablets; caplets; capsules, such as soft clastic gelatin capsules; cachets; troches; lozenges; dispersions; suppositories; powders; aerosols (*e.g.,* nasal sprays or inhalers); gels; liquid dosage forms suitable for oral or mucosal administration to a patient, including suspensions (*e.g.,* aqueous or non-aqueous liquid suspensions, oil-in-water emulsions, or a water-in-oil liquid emulsions), solutions, and elixirs; liquid dosage forms suitable for parenteral administration to a patient; and sterile solids (*e.g.,* crystalline or amorphous solids) that can be reconstituted to provide liquid dosage forms suitable for parenteral administration to a patient.

The composition, shape, and type of dosage forms provided herein will typically vary depending on their use. For example, a dosage form used in the acute treatment of a disease may contain larger amounts of one or more of the active agents it comprises than a dosage form used in the chronic treatment of the same disease. Similarly, a parenteral dosage form may contain smaller amounts of one or more of the active agents it comprises than an oral dosage form used to treat the same disease. These and other ways in which specific dosage forms will vary from one another will be readily apparent to those skilled in the art. *See, e.g.,* Remington's Pharmaceutical Science, 18th ed., Mack Publishing, Easton PA(1990).

Typical pharmaceutical compositions and dosage forms comprise one or more excipients. Suitable excipients are well known to those skilled in the art of pharmacy, and non-limiting examples of suitable excipients are provided herein. Whether a particular excipient is suitable for incorporation into a pharmaceutical composition or dosage form depends on a variety of factors well known in the art including, but not limited to, the way in which the dosage form will be administered to a patient. For example, oral dosage forms such as tablets may contain excipients not suited for use in parenteral dosage forms. The suitability of a particular excipient may also depend on the specific active ingredients in the dosage form. For example, the decomposition of some active ingredients may be accelerated by some excipients such as lactose, or when exposed to water. Active ingredients that comprise primary or secondary amines are particularly susceptible to such accelerated decomposition. Consequently, provided herein are pharmaceutical compositions and dosage forms that contain little, if any, lactose other mono- or disaccharides. As used herein, the term "lactose-free" means that the amount of lactose present, if any, is insufficient to substantially increase the degradation rate of an active ingredient.

Lactose-free compositions can comprise excipients that arc well known in the art and are listed, for example, in the U.S. Pharmacopeia (USP) 25-NF20 (2002). In general, lactose-free compositions comprise active ingredients, a binder/filler, and a lubricant in pharmaceutically compatible and pharmaceutically acceptable amounts. Preferred lactose-free dosage forms comprise active ingredients, microcrystalline cellulose, pre-gelatinized starch, and magnesium stearate.

Also provided herein are anhydrous pharmaceutical compositions and dosage forms comprising active ingredients, since water can facilitate the degradation of some compounds. For example, the addition of water (*e.g.,* 5%) is widely accepted in the pharmaceutical arts as a means of simulating long-term storage in order to determine characteristics such as shelf-life or the stability of formulations over time. *See, e.g.,* Jens T. Carstenscn, Drug Stability: Principles & Practice, 2d. Ed., Marcel Dekker, NY, NY, 1995, pp. 379-80. In effect, water and heat accelerate the decomposition of some compounds. Thus, the effect of water on a formulation can be of great significance since moisture and/or humidity are commonly encountered during manufacture, handling, packaging, storage, shipment, and use of formulations.

Anhydrous pharmaceutical compositions and dosage forms can be prepared using anhydrous or low moisture containing ingredients and low moisture or low humidity conditions. Pharmaceutical compositions and dosage forms that comprise lactose and at least one active ingredients that comprises a primary or secondary amine are preferably anhydrous if substantial contact with moisture and/or humidity during manufacturing, packaging, and/or storage is expected.

An anhydrous pharmaceutical composition should be prepared and stored such that its anhydrous nature is maintained. Accordingly, anhydrous compositions are preferably packaged using materials known to prevent exposure to water such that they can be included in suitable formulary kits. Examples of suitable packaging include, but are not limited to, hermetically scaled foils, plastics, unit dose containers (*e.g.,* vials), blister packs, and strip packs.

Also provided herein are pharmaceutical compositions and dosage forms that comprise one or more compounds that reduce the rate by which an active ingredient will decompose. Such compounds, which are referred to herein as "stabilizers," include, but arc not limited to, antioxidants such as ascorbic acid, pH buffers, or salt buffers.

Like the amounts and types of excipients, the amounts and specific types of active ingredients in a dosage form may differ depending on factors such as, but not limited to, the route by which it is to be administered to patients. In certain embodiments, dosage forms provided herein comprise a PDE4 modulator, or a pharmaceutically acceptable salt, solvate, stereoisomer, or prodrug thereof, in an amount of from about 1 to about 10,000 mg. Typical dosage forms comprise a PDE4 modulator, or a pharmaceutically acceptable salt, solvate, stereoisomer, or prodrug thereof, in an amount of about 1, 2, 5, 10, 25, 50, 100, 200, 400, 800, 1,200, 2,500, 5,000 or 10,000 mg. In a particular embodiment, a preferred dosage form comprises 3-(3,4-dimethoxy-phenyl)-3-(1-oxo-1,3-dihydro-isoindol-2-yl)-propionamide in an amount of about 400, 800 or 1,200 mg. In certain embodiments, dosage forms comprise the second active agent in an amount of form about 1 to about 3,500 mg, from about 5 to about 2,500 mg, from about 10 to about 500 mg, or from about 25 to about 250 mg. Of course, the specific amount of the second active agent will depend on the specific agent used, the type of disease or disorder being treated or managed, and the amount(s) of PDE4 modulators and any optional additional active agents concurrently administered to the patient.

### 4.4.1 Oral Dosage Forms

Pharmaceutical compositions that are suitable for oral administration can be presented as discrete dosage forms, such as, but are not limited to, tablets (*e.g.,* chewable tablets), caplets, capsules, and liquids (*e.g.,* flavored syrups). Such dosage forms contain predetermined amounts of active agents, and may be prepared by methods of pharmacy well known to those skilled in the art. *See generally,* Remington's Pharmaceutical Sciences, 18th ed., Mack Publishing, Easton PA (1990).

Oral dosage forms provided herein may be prepared by combining the active ingredients in an intimate admixture with at least one excipient according to conventional pharmaceutical compounding techniques. Excipients can take a wide variety of forms depending on the form of preparation desired for administration. For example, excipients suitable for use in oral liquid or aerosol dosage forms include, but are not limited to, water, glycols, oils, alcohols, flavoring agents, preservatives, and coloring agents. Examples of excipients suitable for use in solid oral dosage forms (*e.g.,* powders, tablets, capsules, and caplets) include, but are not limited to, starches, sugars, micro-crystalline cellulose, diluents, granulating agents, lubricants, binders, and disintegrating agents.

Because of their ease of administration, tablets and capsules represent the most advantageous oral dosage unit forms, in which case solid excipients are employed. In some embodiments, the dosage form can be a rapid dissolving oral tablet or film, which dissolves quickly after getting in contact with saliva. Such dosage forms are particularly useful for children and elderly, and methods of making such dosage forms arc well-known in the art.

If desired, tablets can be coated by standard aqueous or nonaqueous techniques. Such dosage forms can be prepared by any of the methods of pharmacy. In general, *Pharmaceutical* compositions and dosage forms are prepared by uniformly and intimately admixing the active ingredients with liquid carriers, finely divided solid carriers, or both, and then shaping the product into the desired presentation if necessary.

For example, a tablet can be prepared by compression or molding. Compressed tablets can be prepared by compressing in a suitable machine the active ingredients in a free-flowing form such as powder or granules, optionally mixed with an excipient. Molded tablets can be made by molding in a suitable machine a mixture of the powdered compound moistened with an inert liquid diluent.

Examples of excipients that can be used in oral dosage forms include, but are not limited to, binders, fillers, disintegrants, and lubricants. Binders suitable for use in pharmaceutical compositions and dosage forms include, but are not limited to, corn starch, potato starch, or other starches, gelatin, natural and synthetic gums such as acacia, sodium alginate, alginic acid, other alginates, powdered tragacanth, guar gum, cellulose and its derivatives (*e.g.,* ethyl cellulose, cellulose acetate, carboxymethyl cellulose calcium, sodium carboxymcthyl cellulose), polyvinyl pyrrolidone, methyl cellulose, pre-gelatinized starch, hydroxypropyl methyl cellulose, (*e.g.,* Nos. 2208, 2906, 2910), microcrystalline cellulose, and mixtures thereof.

Suitable forms of microcrystalline cellulose include, but are not limited to, the materials sold as AVICEL-PH-101, AVICEL-PH-103 AVICEL RC-581, AVICEL-PH-105 (available from FMC Corporation, American Viscose Division, Avicel Sales, Marcus Hook, PA), and mixtures thereof. An specific binder is a mixture of microcrystalline cellulose and sodium carboxymcthyl cellulose sold as AVICEL RC-581. Suitable anhydrous or low moisture excipients or additives include AVICEL-PH-103™ and Starch 1500 LM.

Examples of fillers suitable for use in the pharmaceutical compositions and dosage forms provided herein include, but are not limited to, talc, calcium carbonate (*e.g.,* granules or powder), microcrystalline cellulose, powdered cellulose, dextrates, kaolin, mannitol, silicic acid, sorbitol, starch, pre-gelatinized starch, and mixtures thereof. The binder or filler in pharmaceutical compositions is typically present in from about 50 to about 99 weight percent of the pharmaceutical composition or dosage form.

Disintegrants arc used in the compositions to provide tablets that disintegrate when exposed to an aqueous environment. Tablets that contain too much disintegrant may disintegrate in storage, while those that contain too little may not disintegrate at a desired rate or under the desired conditions. Thus, a sufficient amount of disintegrant that is neither too much nor too little to detrimentally alter the release of the active ingredients should be used to form solid oral dosage forms. The amount of disintegrant used varies based upon the type of formulation, and is readily discernible to those of ordinary skill in the art. Typical pharmaceutical compositions comprise from about 0.5 to about 15 weight percent of disintegrant, preferably from about 1 to about 5 weight percent of disintegrant.

Disintegrants that can be used in pharmaceutical compositions and dosage forms provided herein include, but are not limited to, agar-agar, alginic acid, calcium carbonate, microcrystalline cellulose, croscarmellose sodium, crospovidone, polacrilin potassium, sodium starch glycolate, potato or tapioca starch, other starches, pre-gelatinized starch, other starches, clays, other algins, other celluloses, gums, and mixtures thereof.

Lubricants that can be used in pharmaceutical compositions and dosage forms provided herein include, but are not limited to, calcium stcarate, magnesium stearate, mineral oil, light mineral oil, glycerin, sorbitol, mannitol, polyethylene glycol, other glycols, stearic acid, sodium lauryl sulfate, talc, hydrogenated vegetable oil (*e.g.,* peanut oil, cottonseed oil, sunflower oil, sesame oil, olive oil, corn oil, and soybean oil), zinc stearate, ethyl oleate, ethyl laureate, agar, and mixtures thereof. Additional lubricants include, for example, a syloid silica gel (AEROSIL200, manufactured by W.R. Grace Co. of Baltimore, MD), a coagulated aerosol of synthetic silica (marketed by Degussa Co. of Plano, TX), CAB-O-SIL (a pyrogenic silicon dioxide product sold by Cabot Co. of Boston, MA), and mixtures thereof. If used at all, lubricants are typically used in an amount of less than about 1 weight percent of the pharmaceutical compositions or dosage forms into which they are incorporated.

In one embodiment, solid oral dosage form comprises PDE4 modulators, anhydrous lactose, microcrystalline cellulose, polyvinylpyrrolidone, stearic acid, colloidal anhydrous silica, and gelatin.

### 4.4.2 Delayed Release Dosage Forms

Active agents can be administered by controlled release means or by delivery devices that are well known to those of ordinary skill in the art. Examples include, but are not limited to, those described in U.S. Patent Nos.: 3,845,770; 3,916,899; 3,536,809; 3,598,123; and 4,008,719, 5,674,533, 5,059,595, 5,591,767, 5,120,548, 5,073,543, 5,639,476, 5,354,556, and 5,733,566, each of which is incorporated herein by reference. Such dosage forms can be used to provide slow or controlled-release of one or more active ingredients using, for example, hydropropylmethyl cellulose, other polymer matrices, gels, permeable membranes, osmotic systems, multilayer coatings, microparticles, liposomes, microspheres, or a combination thereof to provide the desired release profile in varying proportions. Suitable controlled-release formulations known to those of ordinary skill in the art, including those described herein, can be readily selected for use with the active ingredients provided herein. Thus, provided herein are single unit dosage forms suitable for oral administration such as, but not limited to, tablets, capsules, gelcaps, and caplets that arc adapted for controlled-release.

All controlled-release pharmaceutical products have a common goal of improving drug therapy over that achieved by their non-controlled counterparts. Ideally, the use of an optimally designed controlled-release preparation in medical treatment is characterized by a minimum of drug substance being employed to cure or control the condition in a minimum amount of time. Advantages of controlled-release formulations include extended activity of the drug, reduced dosage frequency, and increased patient compliance. In addition, controlled-release formulations can be used to affect the time of onset of action or other characteristics, such as blood levels of the drug, and can thus affect the occurrence of side (*e.g.,* adverse) effects.

Most controlled-release formulations are designed to initially release an amount of drug (active ingredient) that promptly produces the desired therapeutic effect, and gradually and continually release of other amounts of drug to maintain this level of therapeutic or prophylactic effect over an extended period of time. In order to maintain this constant level of drug in the body, the drug must be released from the dosage form at a rate that will replace the amount of drug being metabolized and excreted from the body. Controlled-release of an active ingredient can be stimulated by various conditions including, but not limited to, pH, temperature, enzymes, water, or other physiological conditions or compounds.

### 4.4.3 Parenteral Dosage Forms

Parenteral dosage forms can be administered to patients by various routes including, but not limited to, subcutaneous, intravenous (including bolus injection), intramuscular, and intraarterial. Because their administration typically bypasses patients' natural defenses against contaminants, parenteral dosage forms are preferably sterile or capable of being sterilized prior to administration to a patient. Examples of parenteral dosage forms include, but arc not limited to, solutions ready for injection, dry products ready to be dissolved or suspended in a pharmaceutically acceptable vehicle for injection, suspensions ready for injection, and emulsions.

Suitable vehicles that can be used to provide parenteral dosage forms are well known to those skilled in the art. Examples include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

Compounds that increase the solubility of one or more of the active ingredients provided herein can also be incorporated into the parenteral dosage forms provided herein. For example, cyclodextrin and its derivatives can be used to increase the solubility of PDE4 modulators and its derivatives. *See, e.g.,* U.S. Patent No. 5,134,127, which is incorporated herein by reference.

### 4.4.4 Topical and Mucosal Dosage Forms

Topical and mucosal dosage forms provided herein include, but are not limited to, sprays, aerosols, solutions, emulsions, suspensions, or other forms known to one of skill in the art. *See, e.g*., Remington's Pharmaceutical Science, 16th and 18th eds., Mack Publishing, Easton PA (1980 & 1990); and Introduction to Pharmaceutical Dosage Forms, 4th cd., Lea & Febiger, Philadelphia (1985). Dosage forms suitable for treating mucosal tissues within the oral cavity can be formulated as mouthwashes or as oral gels.

Suitable excipients (*e.g.,* carriers and diluents) and other materials that can be used to provide topical and mucosal dosage forms are well known to those skilled in the pharmaceutical arts, and depend on the particular tissue to which a given pharmaceutical composition or dosage form will be applied. Some examples of excipients include, but arc not limited to, water, acetone, ethanol, ethylene glycol, propylene glycol, butane-1,3-diol, isopropyl myristate, isopropyl palmitate, mineral oil, and mixtures thereof to form solutions, emulsions or gels, which are non-toxic and pharmaceutically acceptable. Moisturizers or humectants can also be added to pharmaceutical compositions and dosage forms if desired. Examples of such additional ingredients are well known in the art. *See, e.g.,* Remington's Pharmaceutical Sciences, 16th and 18th eds., Mack Publishing, Easton PA (1980 & 1990).

The pH of a pharmaceutical composition or dosage form may also be adjusted to improve delivery of one or more active ingredients. Similarly, the polarity of a solvent carrier, its ionic strength, or tonicity can be adjusted to improve delivery. Compounds such as stearates can also be added to pharmaceutical compositions or dosage forms to advantageously alter the hydrophilicity or lipophilicity of one or more active ingredients so as to improve delivery. In this regard, stearates can serve as a lipid vehicle for the formulation, as an emulsifying agent or surfactant, and as a delivery-enhancing or penetration-enhancing agent. Different salts or solvates of the active ingredients can be used to further adjust the properties of the resulting composition.

### 4.4.5 Kits

In certain embodiments, active ingredients provided herein are not administered to a patient at the same time or by the same route of administration. Thus, provided herein are kits which, when used by the medical practitioner, can simplify the administration of appropriate amounts of active ingredients to a patient.

In one embodiment, the kit comprises a dosage form of a PDE4 modulator, or a pharmaceutically acceptable salt, solvate, hydrate, clathrate, stereoisomer, or prodrug thereof. Kits can further comprise additional active agents or a combination thereof. Examples of the additional active agents include, but are not limited to, those therapeutics discussed herein (*see, e.g.,* section 4.3).

Kits can further comprise devices that are used to administer the active ingredients. Examples of such devices include, but are not limited to, syringes, drip bags, patches, and inhalers.

Kits can further comprise pharmaceutically acceptable vehicles that can be used to administer one or more active ingredients. For example, if an active ingredient is provided in a solid form that must be reconstituted for parenteral administration, the kit can comprise a sealed container of a suitable vehicle in which the active ingredient can be dissolved to form a particulate-free sterile solution that is suitable for parenteral administration. Examples of pharmaceutically acceptable vehicles include, but are not limited to: Water for Injection USP; aqueous vehicles such as, but not limited to, Sodium Chloride Injection, Ringer's Injection, Dextrose Injection, Dextrose and Sodium Chloride Injection, and Lactated Ringer's Injection; water-miscible vehicles such as, but not limited to, ethyl alcohol, polyethylene glycol, and polypropylene glycol; and non-aqueous vehicles such as, but not limited to, corn oil, cottonseed oil, peanut oil, sesame oil, ethyl oleate, isopropyl myristate, and benzyl benzoate.

### 5. EXAMPLES

Certain embodiments are illustrated by the following non-limiting examples.

### 5.1 PHARMACOLOGY STUDIES

Mice or rabbits are aerosol infected by *M*. *tuberculoses* according to the following procedures. In a control group, upon infection by *M. tuberculosis*, the animals are treated with an antibiotic agent (*e.g.,* isoniazid or rifampin). In a second group, the following regimens of PDE4 modulator administration are used: 1) where the animals are pretreated with a PDE4 modulator prior to the infection, followed by antibiotic administration upon infection; 2) where the PDE4 modulator treatment is simultaneous with the infection, followed by antibiotic administration upon infection; and 3) where the animals are treated with a PDE4 modulator after the infection. In the subgroup where the animals are treated with a PDE4 modulator after the infection, the PDE4 modulator is administered before, simultaneously with, or after the treatment by the antibiotic agent.

Acceleration of isoniazid- or rifampin-mediated clearance of *M*. *tuberculosis* is determined under each of the above conditions, and host immune parameters that correlate the changes in *M*. *tuberculosis* physiology that are associated with improved responsiveness to antibiotic treatment, less tissue damage, and markedly less granulomas during and after antibiotic treatment are identified.

For example, determination as to whether enhanced killing of *M. tuberculosis* occurs because of a shift in the metabolic state of the total bacillary population or a shift in the relative proportion of organisms in varying metabolic states is made using lungs from *M. tuberculosis* infected animals.

All of the patents, patent applications and publications referred to in this application are incorporated herein in their entireties. Moreover, citation or identification of any reference in this application is not an admission that such reference is available as prior art. The full scope is better understood with reference to the attached claims.

The application discloses inter alia following items:
1. A method of treating, preventing, or managing tuberculosis comprising administering to a subject a therapeutically or prophylactically effective amount of a PDE4 modulator, or a pharmaceutically acceptable salt, solvate, or stereoisomer thereof, in combination with an anti-tuberculosis agent.
2. The method of item 1, wherein the anti-tuberculosis agent is isoniazid, rifampin, pyrazinamide, streptomycin, ethambutol, capreomycin, ethionamide, cycloserine, levafloxacin, ciprofloxacin, amikacin, moxifloxicin, p-aminosalicylic acid, kanamycin, viomycin, enciomycin, protionamide, rifabutin, clarithromycin, linezolid, thioacetazone, arginine, vitamin B, or a corticosteroid.
3. The method of item 2, wherein the anti-tuberculosis agent is isoniazid or rifampin.
4. The method of item 1, wherein the PDE4 modulator is (+)-2-[1-(3-ethoxy-4-methoxyphenyl)-2-methylsulfonylethyl]-4-acetylaminoisoindoline-1,3-dione.
5. The method of item 1, wherein the PDE4 modulator is cyclopropyl-N-{2-[(1S)-1-(3-ethoxy-4-methoxyphenyl)-2-(methylsulfonyl)ethyl]-3-oxoisoindolin-4-yl}carboxamide.
6. The method of item 1, wherein the PDE4 modulator is cyclopropanecarboxylic acid {2-[1-(3-ethoxy-4-methoxy-phenyl)-2-methanesulfonyl-ethyl]-3-oxo-2,3-dihydro-1*H*-isoindol-4-yl}-amide.
7. The method of item 1, wherein the PDE4 modulator is 3-(3,4-dimethoxyphenyl)-3-(1-oxo-1,3-dihydroisoindol-2-yl)propionic acid methyl ester.
8. The method of item 1, wherein the PDE4 modulator is of formula (I): 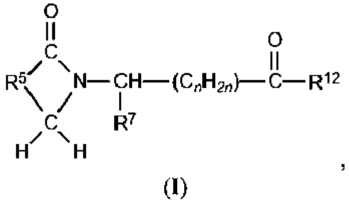 or a pharmaceutically acceptable salt, solvate, or stereoisomer thereof, wherein:
   n has a value of 1, 2, or 3;
   R⁵ is o-phenylene, unsubstituted or substituted with 1 to 4 substituents each selected independently from the group consisting of nitro, cyano, trifluoromethyl, carbcthoxy, carbomcthoxy, carbopropoxy, aectyl, carbamoyl, acetoxy, carboxy, hydroxy, amino, alkylamino, dialkylamino, acylamino, alkyl of 1 to 10 carbon atoms, alkyl of 1 to 10 carbon atoms, and halo;
   R⁷ is (i) phenyl or phenyl substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, (ii) benzyl unsubstituted or substituted with 1 to 3 substituents selected from the group consisting of nitro, cyano, trifluoromethyl, carbothoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, (iii) naphthyl, and (iv) benzyloxy;
   R¹² is -OH, alkoxy of 1 to 12 carbon atoms, or 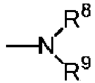
   R⁸ is hydrogen or alkyl of 1 to 10 carbon atoms; and
   R⁹ is hydrogen, alkyl of 1 to 10 carbon atoms, -COR¹⁰, or -SO₂R¹⁰, wherein R¹⁰ is hydrogen, alkyl of 1 to 10 carbon atoms, or phenyl.
9. The method of item 1, wherein the PDE4 modulator is of formula (II): 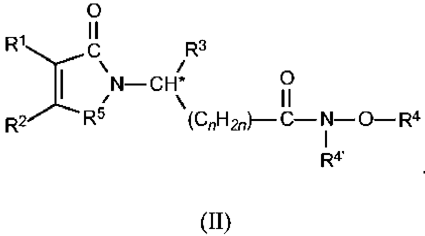 or a pharmaceutically acceptable salt, solvate, or stereoisomer thereof, wherein:
   each of R¹ and R², when taken independently of each other, is hydrogen, lower alkyl, or R¹ and R², when taken together with the depicted carbon atoms to which each is bound, is *o*-phenylene, *o*-naphthylene, or cyclohexene-1,2-diyl, unsubstituted or substituted with 1 to 4 substituents each selected independently from the group consisting of nitro, cyano, trifluoromethyl, carbethoxy, carbomcthoxy, carbopropoxy, acetyl, carbamoyl, acetoxy, carboxy, hydroxy, amino, alkylamino, dialkylamino, acylamino, alkyl of 1 to 10 carbon atoms, alkoxy of 1 to 10 carbon atoms, and halo;
   R³ is phenyl substituted with from one to four substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, alkylthio of 1 to 10 carbon atoms, benzyloxy, cycloalkoxy of 3 to 6 carbon atoms, C₄-C₆-cycloalkylidenemethyl, C₃-C₁₀-alkylidenemethyl, indanyloxy, and halo;
   R⁴ is hydrogen, alkyl of 1 to 6 carbon atoms, phenyl, or benzyl;
   R^{4'} is hydrogen or alkyl of 1 to 6 carbon atoms;
   R⁵ is -CH₂-, -CH₂-CO-,-SO₂-,-S-, or -NHCO-; and
   n has a value of 0, 1, or 2.
10. The method of item 1, wherein the PDE4 modulator is of formula (III): 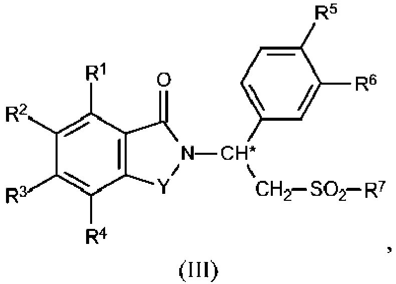 or a pharmaceutically acceptable salt, solvate, or stereoisomer thereof, wherein:
   the carbon atom designated * constitutes a center of chirality;
   Y is C=O, CH2, SO₂, or CH₂C=O;
   each of R¹, R², R³, and R⁴, independently of the others, is hydrogen, halo, alkyl of 1 to 4 carbon atoms, alkoxy of 1 to 4 carbon atoms, nitro, cyano, hydroxy, or -NR⁸R⁹; or any two of R¹, R², R³, and R⁴ on adjacent carbon atoms, together with the depicted phenylene ring are naphthylidene;
   each of R⁵ and R⁶, independently of the other, is hydrogen, alkyl of 1 to 4 carbon atoms, alkoxy of 1 to 4 carbon atoms, cyano, or cycloalkoxy of up to 18 carbon atoms;
   R⁷ is hydroxy, alkyl of 1 to 8 carbon atoms, phenyl, benzyl, or NR^{8'}R^{9'};
   each of R⁸ and R⁹ taken independently of the other is hydrogen, alkyl of 1 to 8 carbon atoms, phenyl, or benzyl, or one of R⁸ and R⁹ is hydrogen and the other is - COR¹⁰ or -SO₂R¹⁰, or R⁸ and R⁹ taken together are tetramethylene, pentamethylene, hexamethylene, or -CH₂CH₂X¹CH₂CH₂- in which X¹ is -O-, -S- or -NH-; and
   each of R^{8'} and R^{9'} taken independently of the other is hydrogen, alkyl of 1 to 8 carbon atoms, phenyl, or benzyl, or one of R^{8'} and R^{9'} is hydrogen and the other is -COR^{10'} or -SO₂R^{10'}, or R^{8'} and R^{9'} taken together are tetramethylene, pentamethylene, hexamethylene, or -CH₂CH₂X²CH₂CH₂- in which X² is -O-, -S-, or -NH-.
11. The method of item 1, wherein the PDE4 modulator, or a pharmaceutically acceptable salt, solvate, or stereoisomer thereof, is administered prior to the *tuberculosis* infection.
12. The method of item 1, wherein the PDE4 modulator, or a pharmaceutically acceptable salt, solvate, or stereoisomer thereof, is administered subsequent to the tuberculosis infection.
13. The method of item 1, wherein PDE4 modulator, or a pharmaceutically acceptable salt, solvate, or stereoisomer thereof, is administered prior to the administration of the anti-tuberculosis agent.
14. The method of item 1, wherein PDE4 modulator, or a pharmaceutically acceptable salt, solvate, or stereoisomer thereof, is administered simultaneously with the anti-tuberculosis agent.
15. The method of item 1, wherein PDE4 modulator, or a pharmaceutically acceptable salt, solvate, or stereoisomer thereof, is administered subsequent to the administration of the anti-tuberculosis agent.
16. The method of item 1, wherein PDE4 modulator, or a pharmaceutically acceptable salt, solvate, or stereoisomer thereof, and the anti-tuberculosis agent is administered using the same route.
17. The method of item 1, wherein PDE4 modulator, or a pharmaceutically acceptable salt, solvate, or stereoisomer thereof, and the anti-tuberculosis agent is administered using different routes.

## Claims

1. A PDE4 modulator for use in a method of treating, preventing, or managing tuberculosis, the method comprising administering to a subject a PDE4 modulator, or a pharmaceutically acceptable salt, solvate, or stereoisomer thereof, in combination with an anti-tuberculosis agent, wherein the PDE4 modulator is:
(1) a compound of formula (I) 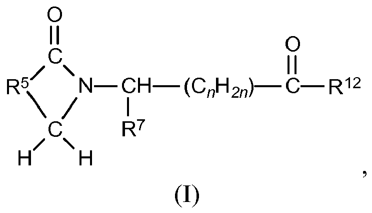 or a pharmaceutically acceptable salt, solvate, or stereoisomer thereof, wherein:
n has a value of 1, 2, or 3;
R⁵ is o-phenylene, unsubstituted or substituted with 1 to 4 substituents each selected independently from the group consisting of nitro, cyano, trifluoromethyl, carbethoxy, carbomethoxy, carbopropoxy, acetyl, carbamoyl, acetoxy, carboxy, hydroxy, amino, alkylamino, dialkylamino, acylamino, alkyl of 1 to 10 carbon atoms, alkyl of 1 to 10 carbon atoms, and halo;
R⁷ is (i) phenyl or phenyl substituted with one or more substituents each selected independently of the other from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, (ii) benzyl unsubstituted or substituted with 1 to 3 substituents selected from the group consisting of nitro, cyano, trifluoromethyl, carboethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, and halo, (iii) naphthyl, and (iv) benzyloxy;
R¹² is -OH, alkoxy of 1 to 12 carbon atoms, or 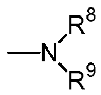
R⁸ is hydrogen or alkyl of 1 to 10 carbon atoms; and
R⁹ is hydrogen, alkyl of 1 to 10 carbon atoms, -COR¹⁰, or -SO₂R¹⁰, wherein R¹⁰ is hydrogen, alkyl of 1 to 10 carbon atoms, or phenyl;
(2) a compound of formula (II) 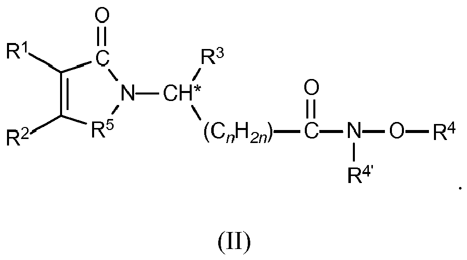 or a pharmaceutically acceptable salt, solvate, or stereoisomer thereof, wherein:
each of R¹ and R², when taken independently of each other, is hydrogen, lower alkyl, or R¹ and R², when taken together with the depicted carbon atoms to which each is bound, is o-phenylene, o-naphthylene, or cyclohexene-1,2-diyl, unsubstituted or substituted with 1 to 4 substituents each selected independently from the group consisting of nitro, cyano, trifluoromethyl, carbethoxy, carbomethoxy, carbopropoxy, acetyl, carbamoyl, acetoxy, carboxy, hydroxy, amino, alkylamino, dialkylamino, acylamino, alkyl of 1 to 10 carbon atoms, alkoxy of 1 to 10 carbon atoms, and halo;
R³ is phenyl substituted with from one to four substituents selected from the group consisting of nitro, cyano, trifluoromethyl, carbethoxy, carbomethoxy, carbopropoxy, acetyl, carbamoyl, acetoxy, carboxy, hydroxy, amino, alkyl of 1 to 10 carbon atoms, alkoxy of 1 to 10 carbon atoms, alkylthio of 1 to 10 carbon atoms, benzyloxy, cycloalkoxy of 3 to 6 carbon atoms, C₄-C₆-cycloalkylidenemethyl, C₃-C₁₀-alkylidenemethyl, indanyloxy, and halo;
R⁴ is hydrogen, alkyl of 1 to 6 carbon atoms, phenyl, or benzyl;
R^{4'} is hydrogen or alkyl of 1 to 6 carbon atoms;
R⁵ is -CH₂-, -CH₂-CO-,-SO₂-,-S-, or -NHCO-; and
n has a value of 0, 1, or 2;
(3) a compound of formula (III) 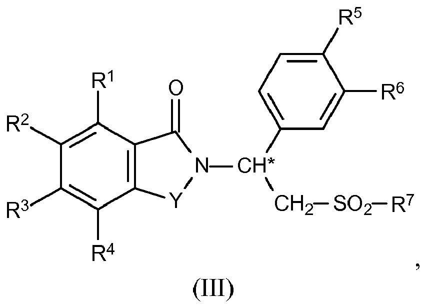 or a pharmaceutically acceptable salt, solvate, or stereoisomer thereof, wherein:
the carbon atom designated * constitutes a center of chirality;
Y is C=O, CH₂, SO₂, or CH₂C=O;
each of R¹, R², R³, and R⁴, independently of the others, is hydrogen, halo, alkyl of 1 to 4 carbon atoms, alkoxy of 1 to 4 carbon atoms, nitro, cyano, hydroxy, or -NR⁸R⁹; or any two of R¹, R², R³, and R⁴ on adjacent carbon atoms, together with the depicted phenylene ring are naphthylidene;
each of R⁵ and R⁶, independently of the other, is hydrogen, alkyl of 1 to 4 carbon atoms, alkoxy of 1 to 4 carbon atoms, cyano, or cycloalkoxy of up to 18 carbon atoms;
R⁷ is hydroxy, alkyl of 1 to 8 carbon atoms, phenyl, benzyl, or NR^{8'}R^{9'};
each of R⁸ and R⁹ taken independently of the other is hydrogen, alkyl of 1 to 8 carbon atoms, phenyl, or benzyl, or one of R⁸ and R⁹ is hydrogen and the other is -COCH₃ or R⁸ and R⁹ taken together are tetramethylene, pentamethylene, hexamethylene, or - CH₂CH₂X¹CH₂CH₂- in which X¹ is -O-, -S- or -NH-; and each of R^{8'} and R^{9'} taken independently of the other is hydrogen, alkyl of 1 to 8 carbon atoms, phenyl, or benzyl, or R^{8'} and R⁹ taken together are tetramethylene, pentamethylene, hexamethylene, or - CH₂CH₂X²CH₂CH₂- in which X² is -O-, -S-, or -NH-; and
each of R^{8'} and R^{9'} taken independently of the other is hydrogen, alkyl of 1 to 8 carbon atoms, phenyl, or benzyl, or one of R^{8'} and R⁹ is hydrogen and the other is -COR^{10'} or -SO₂R^{10'}, or R^{8'} and R^{9'} taken together are tetramethylene, pentamethylene, hexamethylene, or -CH₂CH₂X²CH₂CH₂- in which X² is -O-, -S-, or -NH-.
(4) ((+)-2-[1-(3-ethoxy-4-methoxyphenyl)-2-methylsulfonylethyl]-4-acetylaminoisoindoline-1,3-dione, or a pharmaceutically acceptable salt, solvate, or stereoisomer thereof;
(5) cyclopropyl-N-{2-[(1S)-1-(3-ethoxy-4-methoxyphenyl)-2-(methylsulfonyl)ethyl]-3-oxoisoindolin-4-yl}carboxamide, or a pharmaceutically acceptable salt, solvate, or stereoisomer thereof;
(6) cyclopropanecarboxylic acid {2-[1-(3-ethoxy-4-methoxy-phenyl)-2-methanesulfonyl-ethyl]-3-oxo-2,3-dihydro-1*H*-isoindol-4-yl}-amide, or a pharmaceutically acceptable salt, solvate, or stereoisomer thereof; or
(7) 3-(3,4-dimethoxyphenyl)-3-(1-oxo-1,3-dihydroisoindol-2-yl)propionic acid methyl ester, or a pharmaceutically acceptable salt, solvate, or stereoisomer thereof;
and wherein the anti-tuberculosis agent is isoniazid or rifampin.

2. The PDE4 modulator for use of claim 1,
wherein the PDE4 modulator, or a pharmaceutically acceptable salt, solvate, or stereoisomer thereof, is administered prior to the tuberculosis infection; or
wherein the PDE4 modulator, or a pharmaceutically acceptable salt, solvate, or stereoisomer thereof, is administered subsequent to the tuberculosis infection.

3. The PDE4 modulator for use of claim 1,
wherein PDE4 modulator, or a pharmaceutically acceptable salt, solvate, or stereoisomer thereof, is administered prior to the administration of the anti-tuberculosis agent; or
wherein PDE4 modulator, or a pharmaceutically acceptable salt, solvate, or stereoisomer thereof, is administered simultaneously with the anti-tuberculosis agent; or
wherein PDE4 modulator, or a pharmaceutically acceptable salt, solvate, or stereoisomer thereof, is administered subsequent to the administration of the anti-tuberculosis agent.

4. The PDE4 modulator for use of claim 1,
wherein PDE4 modulator, or a pharmaceutically acceptable salt, solvate, or stereoisomer thereof, and the anti-tuberculosis agent is administered using the same route; or wherein PDE4 modulator, or a pharmaceutically acceptable salt, solvate, or stereoisomer thereof, and the anti-tuberculosis agent is administered using different routes.
